Europäisches Patentamt

European Patent Office

Office européen des brevets

(19)

(11) **EP 0 474 691 B1**

(12) ## EUROPEAN PATENT SPECIFICATION

(45) Date of publication and mention
of the grant of the patent:
**13.11.1996 Bulletin 1996/46**

(21) Application number: **90908270.3**

(22) Date of filing: **31.05.1990**

(51) Int Cl.6: **C12P 21/08**, **A61K 39/395**

(86) International application number:
**PCT/GB90/00840**

(87) International publication number:
**WO 90/15152 (13.12.1990 Gazette 1990/28)**

(54) **MONOCLONAL ANTIBODIES FOR INDUCING TOLERANCE**

MONOKLONALE ANTIKÖRPER ZUR INDUZIERUNG VON TOLERANZ

ANTICORPS MONOCLONAUX POUR SUSCITER UNE TOLERANCE

(84) Designated Contracting States:
**AT BE CH DE DK ES FR GB IT LI LU NL SE**

(30) Priority: **31.05.1989 GB 8912497**

(43) Date of publication of application:
**18.03.1992 Bulletin 1992/12**

(73) Proprietor: **THE WELLCOME FOUNDATION
LIMITED
Greenford, Middlesex UB6 0NN (GB)**

(72) Inventors:
• **Cobbold, Stephen Paul
Oxford OX1 3RE (GB)**
• **Waldmann, Herman
Oxford OX1 3RE (GB)**

(74) Representative: **Marchant, James Ian et al
Elkington and Fife,
Prospect House,
8 Pembroke Road
Sevenoaks, Kent TN13 1XR (GB)**

(56) References cited:
• TRANSPLANTATION PROCEEDINGS, vol. XIX,
no. 5, October 1987, Grune & Stratton Inc., New
York, NY (US); M. JONKER et al., pp. 4308-4314
• IMMUNOLOGICAL & CELLULAR BIOLOGY, vol.
67, no. 1, January 1989, Adelaide (AU); B.
CHARLTON et al., pp. 1-7
• JOURNAL OF IMMUNOLOGY, vol. 142, no. 5, 01
March 1989, American Association of
Immunologists, Baltimore, MD (US); N.L.
CARTERON et al., pp. 1470-1475
• EUROPEAN JOURNAL OF IMMUNOLOGY, vol.
17, 1987, VCH VerlagsgesmbH., Weinheim (DE);
S. QIN et al., pp. 1159-1165

EP 0 474 691 B1

**Description**

This invention relates to tolerance induction using monoclonal antibodies.

Tolerance to foreign antigen or tissue, or self antigen or tissue is a state whereby an otherwise normal, mature immune system is specifically unable to respond aggressively to that antigen/tissue which it therefore treats like a normal (non-diseased) body tissue/component, yet at the same time it can respond aggressively to foreign or diseased antigens/tissues to which it has not been specifically made tolerant by the natural process of self tolerance or by therapeutic tolerance induction procedures. A test for tolerance usually requires a demonstration that the tolerant individual fails to become immune to the specific antigen/tissue when one or preferably more attempts to immunize are made at a later time when the same individual can be shown to respond to an irrelevant antigen/tissue.

Monoclonal antibodies (mAb) against the murine CD4 (L3T4) antigen have proven to be potent immunosuppressive agents for the control of humoral immunity, transplant rejection and autoimmunity. In addition, CD4 mAbs have been shown to create a tolerance-permissive environment in vivo with which can be achieved tolerance to certain soluble protein antigens as well as transplantation antigens. However, the mechanism(s) by which CD4 mAbs produce these effects are still not clear. In most previous reports, immunosuppression was obtained under conditions that depleted target cells in vivo. A simple interpretation was that the immune suppression so achieved was due to the absence of CD4 T cells.

On the other hand, in vitro work has demonstrated that CD4 (and CD8) mAbs could affect lymphocyte functions simply through binding to the antigen on the cell surface without cell lysis.

Qin et al, Eur. J. Immunol, 17 1159-1165 (1987) investigated the use of a pair of depleting anti-CD4 monoclonal antibodies which recognised different epitopes of the antigen and observed an immunosuppressive effect at sub-lytic doses. Other workers have worked with depleting anti-CD4 antibodies but in the form of $F(ab')_2$ fragments which would not be capable of cellular depletion. Thus Gutstein & Wofsy, J. Immunol, 137, 3414-3419 (1986) looked at the effect of $F(ab')_2$ fragments of an anti-L3T4 antibody on the response of mice to bovine serum albumins and concluded that the ability of the anti-L3T4 antibody to inhibit humoral immunity was not dependent on the depletion of L3T4$^+$ cells. Benjamin et al, Eur. J. Immunol., 18, 1079-1088 (1988) worked with $F(ab')_2$ fragments of a depleting anti-CD4 antibody and showed that they were able to provide a tolerogenic milieu in respect of the antigen human IgG in the mouse without cellular depletion. Carteron et al J. Immunol., 140, 713-716 (1988) used $F(ab')_2$ fragments of a depleting anti-L3T4 antibody and found that these fragments could induce tolerance to an antigen (a rat monoclonal antibody to chicken ovalbumin) in the mouse independent of the ability of the antibody to deplete L3T4$^+$ cells. It is not possible to predict from this work using depleting anti-CD4 antibodies but in a manner which would not lead to cellular depletion that intact non-depleting anti-CD4 antibodies used at appropriate doses without cellular depletion would induce tolerance.

Qin et al, J. Exp. Med., 169, 779-794 (1989) set out to induce "classical transplantation tolerance" in adult animals by means of bone marrow transplantation and used a combination of CD4 and CD8 monoclonal antibodies (both depleting and non-depleting) in order to enable the bone marrow transplant to become established. There is no suggestion that the antibodies themselves were inducing tolerance.

Charlton and Mandel, Immunol. Cell. Biol., 67, 1-7 (1989) used anti-L3T4 monoclonal antibodies to induce tolerance to aggregated human IgG and ovalbumin. The authors claim to have used both depleting and non-depleting antibodies, however the effect of the antibodies on L3T4$^+$ T-cells was investigated only in the spleen and thymus. The fact that an antibody does not deplete T-cells in the spleen and thymus does not provide any evidence of the effect of the antibody on circulating T-cells.

Jonker et al Transplantation Proceedings, XIX(5), 4308-4314 (1987) investigated the question of whether the immunosuppressive effects of CD4 and CD8 antibodies were related to the epitope specificity of the antibodies and their effects on CD4 and CD8 subsets. There is no mention of the induction of immunological tolerance and no suggestion of the use of a non-depleting CD4 antibody to induce tolerance to an antigen.

Previous studies have used antibodies that deplete CD4 cells. We have now found that non-depleting CD4 antibodies, optionally together with CD8 antibodies, can also produce tolerance to foreign immunoglobulins, bone marrow and skin grafts. Indeed, this observation has general applicability to all antigens. Further, we have found that administration of a depleting CD4 mAb and/or a depleting CD8 mAb prior to administration of the non-depleting mAbs can be beneficial in creating a tolerance-permissive environment.

According to one aspect, the present invention provides the use of a non-depleting anti-CD4 monoclonal antibody for the manufacture of a medicament for the induction of a state of immunological tolerance to an antigen by a method which comprises administering said non-depleting anti-CD4 monoclonal antibody to a subject together with a non-depleting anti-CD8 monoclonal antibody to induce an immunological tolerance permissive environment within said subject by means of said antibodies in the presence of said antigen.

According to another aspect, the present invention provides the use of a non-depleting anti-CD8 monoclonal antibody for the manufacture of a medicament for the induction of a state of immunological tolerance to an antigen by a method which comprises administering said non-depleting anti-CD8 monoclonal antibody to a subject together with a non-depleting anti-CD4 monoclonal antibody to induce a tolerance permissive environment within said subject by means of said antibodies in the presence of said antigen.

According to a further aspect, the present invention provides the use of a non-depleting anti-CD4 monoclonal antibody as the whole antibody for the manufacture of a medicament for the induction of a state of immunological tolerance to an antigen selected from autoantigens, transplantation antigens and immunoglobulins by a method which comprises administering said non-depleting anti-CD4 monoclonal antibodies to a subject to induce an immunological tolerance permissive environment within said subject by means of said antibody in the presence of said antigen.

The present invention also provides a pharmaceutical composition comprising from 1 to 400 mg per dose of a non-depleting anti-CD4 antibody or a non-depleting anti-CD8 antibody together with a pharmaceutically acceptable carrier or diluent in a form suitable for administration to a human subject.

By administering non-depleting CD4 and CD8 mAbs together, tolerance can be conferred to a primary antigen in a patient. The CD4 and CD8 mAbs can be used to treat autoimmune diseases and to prevent graft rejection without the need for long term immunosuppressive chemotherapy. Tolerance to a graft such as an organ graft or bone marrow transplantation can be achieved.

There are advantages of using non-depleting mAbs in vivo. For example, injection of a short course of non-depleting mAbs allows quicker recovery of competent cells from blockade and therefore may lessen the risk of opportunistic infection and other complications due to immune deficiency (e.g. leukemia relapse after T-depleted bone-marrow transplantation) following mAb treatment. In addition, it is now known that CD4 cells can be further divided into different functional subsets, some of which may be involved in immune regulation. Bulk elimination of T cells may of course result in immune suppression, but it may also destroy an possible influence of CD4+ regulatory cells. Therefore, a more subtle manipulation may be more beneficial for guiding the immune system to a desired state.

Tolerance may preferably be attained by administering first to a patient depleting CD4 and/or CD8 mAbs.

A combination of non-depleting CD4 and CD8 mAbs can be used to induce tolerance to any antigen without the need for other immunosuppressive agents. A non-depleting mAb is a mAb which depletes fewer than 50%, for example from 10 to 25% and preferably less than 10% of target cells in vivo. They may be used to induce tolerance to a Class I antigen or to a Class II antigen or to an antigen presented by a Class I or Class II antigen. They may be used to induce tolerance to both antigens. In the case of a transplant, for example, Class I and Class II major histocompatibility (MHC) antigens and non-MHC or minor histocompatibility (minors) antigens may be presented. Apart from transplantation antigens, the present invention can be used to induce tolerance to globular proteins, glycoproteins such as immunoglobulins, materials carried on particles such as pollen proteins, polypeptides intended for therapeutic use such as interferon, interleukin-2 or tumour necrosis factor, or hormone replacements such as leutinizing hormone, its analogues and antagonists. Further specific antigens to which tolerance can be conferred include synthetic peptide analogues of protein therapeutic agents which are used for receptor block aid and alloantigens. It is alloantigens which are considered to be responsible for rejection of foreign tissue in tissue transplants or skin grafts.

The mAbs which may be used are mAbs which are specific for the CD4 cell surface antigen (CD4 mAb) or for the CD8 cell surface antigen (CD8 mAb). By CD4 and Cd8 mAbs we mean not only mAbs specific for the human CD4 and CD8 surface antigens but also mAbs specific for the corresponding surface antigens in other species such as the L3T4 antigen in mice which is the murine equivalent of the human CD4 antigen. The mAbs are typically of the $IgG_2$ Class such as rat $IgG_{2a}$, mouse $IgG_{2b}$ or human $IgG_2$, but may be human $IgG_4$. mAb fragments comprising the antibody binding site may be used, such as Fab and $F(ab)_2$ fragments.

The CD4 and CD8 mAbs are administered together to a host. They may be administered as part of the same formulation or as separate formulations. Typically both mAbs are administered from 1 to 7 times a week, preferably from 1 to 4 times a week, for example three times a week, for from 2 to 4 weeks, preferably for 3 weeks. An effective amount of the non-depleting mAbs is given. Testing for saturating amounts of antibody in serum should indicate that sufficient antibody is present. Enough of each non-depleting mAb is consequently administered to induce a tolerance-permissive environment in a subject under treatment. The CD4 and CD8 cells can thus be blocked.

The amount of non-depleting CD4 mAb and of non-depleting CD8 mAb administered to a patient depends upon a variety of factors including the age and weight of a patient, the condition which is being treated and the antigen(s) to which it is desired to induce tolerance. In a model mouse system from 1μg to 2mg, preferably from 400μg to 1mg, of a mAb is administered at any one time. In humans from 1 to 400mg, such as from 3 to 30mg, for example from 5 to 20mg, of antibody may be given. A CDlla mAb, a non-depleting mAb, may be used in addition to CD4 and CD8 mAbs or in place of either or both of the CD4 or CD8 mAbs.

A foreign antigen(s) to which it is desired to induce tolerance can be administered to a host from up to 5 days before a course of non-depleting CD4 and CD8 mAbs is commenced to up to 5 days or even 2 to 3 weeks after the course has been completed. Generally, however, an antigen is administered within the first 14 days of the course commencing, typically within 7 days of the course commencing. The antigen can be given at the time the course of CD4 and CD8 mAb treatment is commenced.

Tolerance can therefore be induced to an antigen in a host by administering non-depleting CD4 and CD8 mAbs and, under cover of the mAbs, the antigen. A patient may be operated on surgically under cover of the non-depleting CD4 and CD8 mAbs to be given a tissue transplant such as an organ graft or a bone marrow transplant. Also, tolerance may be induced to an antigen already possessed by a subject. Long term specific tolerance can be induced to a self antigen or antigens in order to treat autoimmune disease such as multiple sclerosis or rheumatoid arthritis. The condition of a patient suffering from autoimmune disease can therefore be alleviated.

Persistant antigen is required to maintain tolerance. A tissue graft, for example, supplies the antigen to maintain tolerance to itself. The same applies to self (auto) antigens in the treatment of autoimmune diseases. In the case of extraneous foreign antigen such as allergens, antigen reminders can be given at regular intervals.

It may be preferable to treat a host with a depleting CD4 mAb and/or a depleting CD8 mAb before commencing treatment with non-depleting mAbs. A depleting mAb is a mAb which depletes more than 50%, for example from 90 to 99%, of target cells in vivo. Depleting antibodies include rat $IgG_{2b}$ or $IgG_1$, mouse $IgG_{2a}$ and human $IgG_1$ and $IgG_3$. A depleting CD4 mAb and/or a depleting CD8 mAb may therefore be used to reduce the relevant population of T cells. The non-depleting mAbs therefore have fewer T cells to work on. Depletion may alternatively be achieved by conventional immunosuppressive therapy such as administration of another mAb such as CAMPATH (Trade Mark) 1, steroids, cyclosporin, ALG (anti-lymphocyte globulin) or irradiation.

The level to which a population of T cells is reduced by a depleting mAb may depend upon the antigen(s) to which it is wished to achieve tolerance. It may be preferable to reduce the T cell population further for difficult antigens, for poorly matched tissue grafts, where recipients have been previously exposed to donor antigens and are primed (for example multiply transfused patients) or in autoimmune diseases where the patients are both primed and undergoing continuous activation of their autoimmune state. A poor tissue match, for example, is where one or more Class I major histocompatibility antigens do not match.

It may therefore be necessary to reduce a population of CD4-positive helper T cells to less than about 70%, for example less than about 50%, 20% or even 10%, of their normal level. The more difficult it is likely to be to achieve tolerance, the greater the amount of depletion it is desirable to achieve. CD8-positive T cells may be depleted in the same way.

The depleting CD4 mAb and/or depleting CD8 mAb is typically administered from 1 to 7 times a week, preferably from 2 to 4 times a week, for example three times a week or once or twice, preferably once, from 1 to 7 days, for example from 1 to 5 days, before commencement of the treatment with non-depleting CD4 and CD8 mAbs. An antigen to which it is desired to induce tolerance may be administered at the same time as, or within 5 days of, administration of the depleting mAb. The amount of depleting mAb which is given depends upon a variety of factors including the level to which it is desired to reduce the relevant population of T cells, the age and weight of a patient, the condition which is being treated and the antigen(s) to which it is desired to induce tolerance. In a mouse model system, dose of from 1μg to 2mg, preferably from 400μg to 1mg, of a depleting mAb may be given. In humans, from 1 to 400mg, such as from 3 to 30mg, for example from 5 to 20mg, of antibody may be given.

The depleting and non-depleting CD4 and CD8 mAbs can be raised in any convenient manner. They may be made by conventional methods of fusing immune rat spleen cells to a rat myeloma cell line such as Y3/Ag 1.2.3. (Clark and Waldmann, chapter 1 of "Monoclonal Antibodies", which is a book edited by P.C.L. Beverley in a series "Methods in Hematology", Longman (Churchill Livingstone), 1986). Any other method may be employed such as by fusing immunised mouse spleen cells with a mouse myeloma, a rat myeloma or human myeloma or by recombinant DNA methodologies.

All mAb administrations are given parenterally, for example intravenously. The mAbs are generally being given by injection or by infusion. For this purpose a mAb is formulated in a pharmaceutical composition containing a pharmaceutically acceptable carrier or diluent. Any appropriate carrier or diluent may be used, for example isotonic saline solution. When specific antigens to which it is wished to confer tolerance are given, these may be administered parenterally, for example intravenously, intramuscularly or subcutaneously. The antigen is typically formulated with a pharmaceutically acceptable carrier or diluent as above.

The respective hybridomas producing mAbs YTS 177.9, YTS 191.1, YTS 105.18 and YTS 169.4 were deposited at the European Collection of Animal Cell Cultures, Porton Down, G.B. on 30 May 1990 under accession numbers ECACC 90053005, ECACC 87072282, ECACC 90053004 and ECACC 87072284.

The following Examples illustrate the invention. In the accompanying drawings:

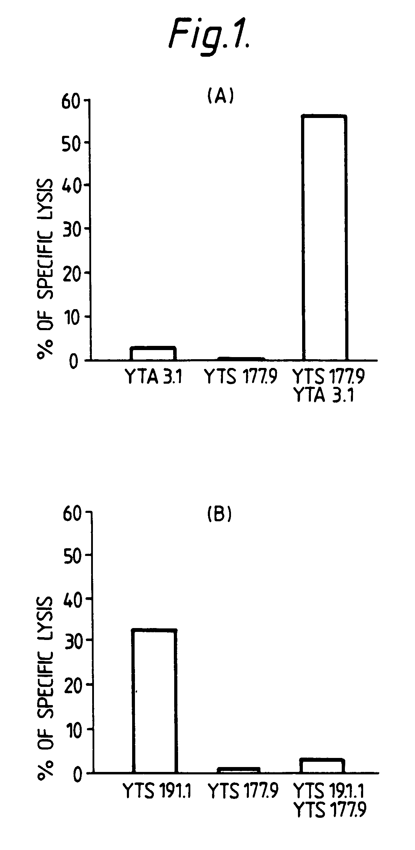

Figure 1 shows synergy and interference of complement lysis by combinations of rIgG2a and rIgG2b Mabs. CBA/Ca thymocytes labelled with $^{51}$Cr (30μCi/ml) were incubated with Mabs as indicated under each bar and 2% guinea pig serum as the complement source. Mab concentration: (A) 5μg/ml; (B) 25μg/ml of the Mabs used.

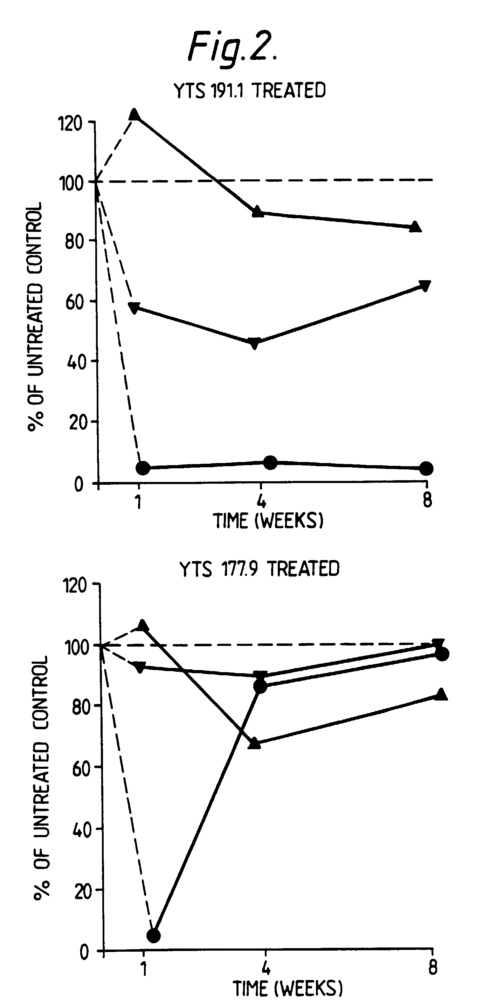

Figure 2 shows the results of injection of rIgG2a CD4 in vivo without T cell depletion. ATX CBA/Ca mice (n=3) were given a single injection of the indicated Mabs (2mg/mouse) and bled at 1, 4 and 8 weeks later. BPL were stained with biotinylated Mabs against CD4 (YTA 3.1 ●), CD8 (YTS 156.7 ▲) and Thy-I (YBM 29.2 ▼), followed by streptavidin-FITC. The results were analysed by flow cytometry and the percentage of each population was calculated using that of the untreated control on the test day as 100%.

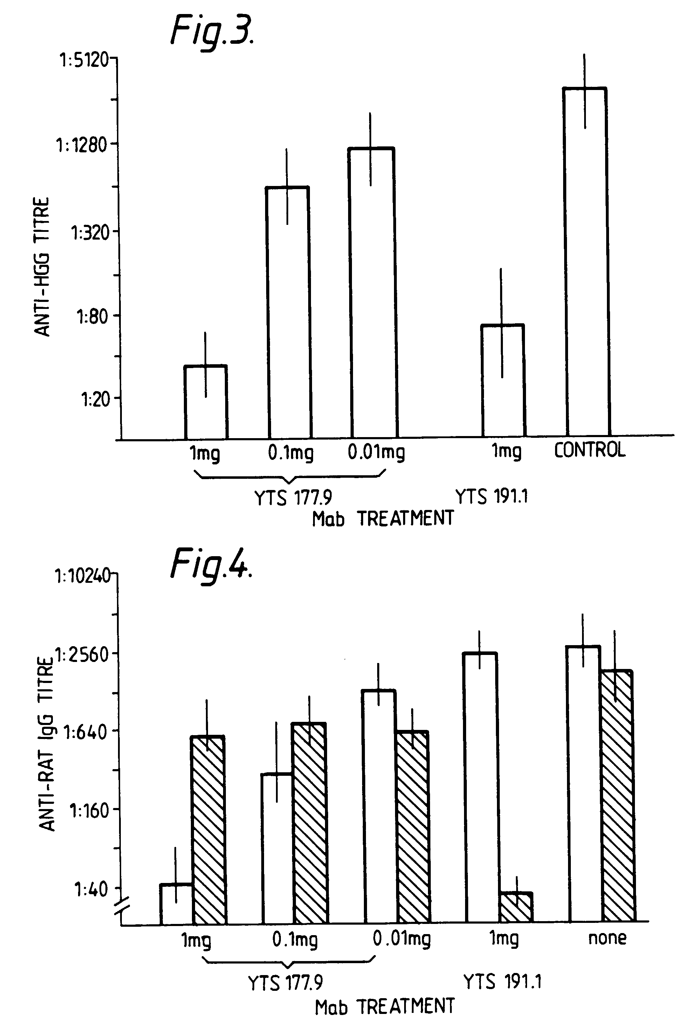

Figure 3 shows how tolerance to HGG was induced by IgG2a CD4 Mab YTS 177.9. Normal CBA/Ca mice were given YTS 177.9 or YTS 191.1, at doses indicated on days -1, 0 and 1. 1 mg heat-aggregated HGG was injected on day 0. The mice were rechallenged with 0.5 mg HGG on days 28 and 35. Control mice received 1 mg of YTS 177.9 as others, but immunized with HGG only on days 28 and 35. Serum anti-HGG titres were measured on day 45 by an ELISA assay.

Figure 4 shows the results of injection of YTS 177.9 to induce specific tolerance to rat IgG2a. Normal CBA/Ca mice were given 3 injections of YTS 177.9 or YTS 191.1 at the indicated doses on 3 consecutive days. 6 weeks later, they were rechallenged with weekly injection of YTH 35.4 (IgG2a rat anti-human) and Campath (Registered Trade Mark) 2 (IgG2b rat anti-human), first in complete, then in incomplete Freunds adjuvant. On the tenth week, they were bled and anti-IgG2a (open-bar) and anti-IgG2b (cross-hatched bar) titres were measured with an ELISA assay using plates coated with HPLC-purified rat IgG2a and IgG2b mabs respectively.

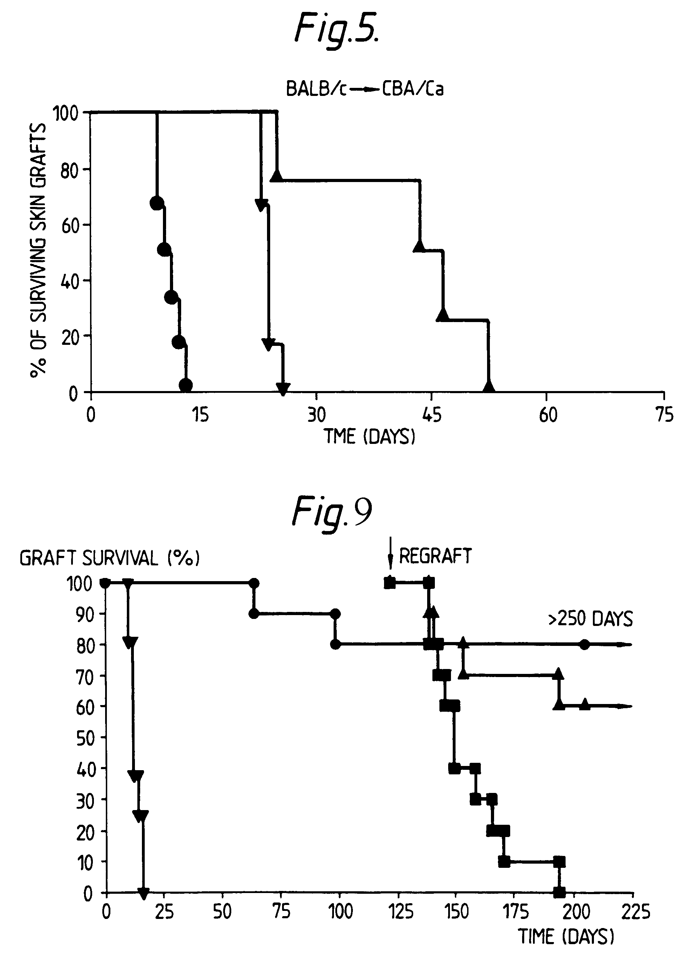

Figure 5 shows prolonged allogeneic skin graft survival after IgG2a CD4 and CD8 Mab treatment. Normal CBA/Ca mice (n=6) were given 3 injections of YTS 177.9 and YTS 105.18 or YTS 191.1 and YTS 169.4 (total antibody, 3 mg/mouse) on days 0, 2 and 4. Full thickness BALB/c tail skin was grafted on day 0. Statistic value by Logrank method: IgG2b Mab treated (▲) v.s. non-treated control (●) p<0.005; IgG2a treated (▼) v.s. control p≤0.001; v.s. IgG2b treated p≤0.03.

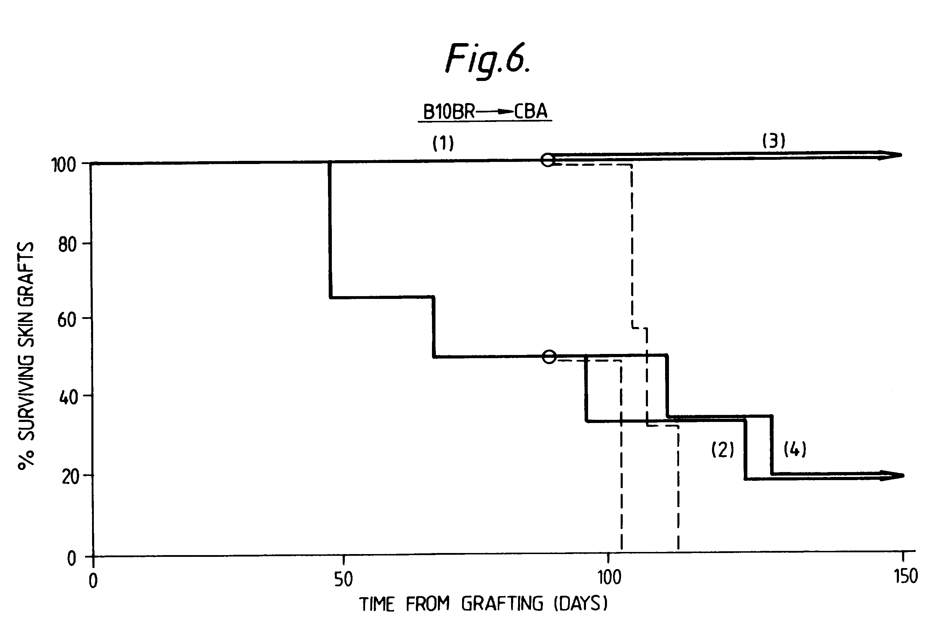

Figure 6 shows tolerance to allogeneic skin grafts by rIgG2a mAbs. Normal CBA/Ca mice were grafted with B10.BR skin and received (1) a mixture of YTS 177.9 and YTS 105.18 on days 0 and 2 (2 mg/mouse total); (2) the same mAbs over 3 weeks (9 mg/mouse total). On day 89, they were regrafted with skin from B10.BR (3 and 4) and B10.D2 (dotted lines).

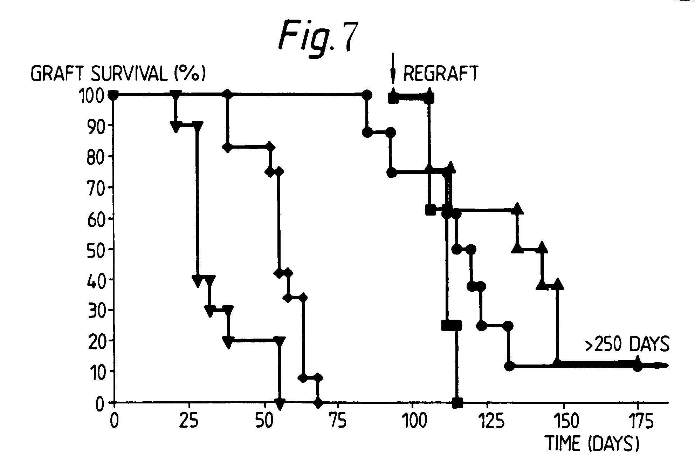

Figure 7 shows allogeneic skin graft survival in mice treated with difference combinations of CD4 and CD8 antibodies. Recipient CBA/Ca mice, in three groups of 8-14, were grafted with BALB/c skin on day 0, and treated with monoclonal antibodies from day 0 to day 2, as described in Example 2. The first group (▼) received only rat $IgG_{2a}$ antibodies, the second (♦) received a cocktail of rat $IgG_{2b}$ antibodies, while the third group (●) was given the combination protocol of rat $IgG_{2b}$ followed by rat $IgG_{2a}$ antibodies. All mice in this third group were given new BALB/c (▲) and B10 (■) skin grafts at day 94 without further antibody.

Analysis of graft survival: $IgG_{2a}$ group MST=28 days versus $IgG_{2b}$ group MST=55 days; P<0.006: Combined protocol group original BALB/c graft MST=121 days compared to $IgG_{2a}$ or $IgG_{2b}$ groups P<0,001: Combined protocol group second BALB/c graft MST=43 days compared to third party B10 graft MST=16 days P<0.04.

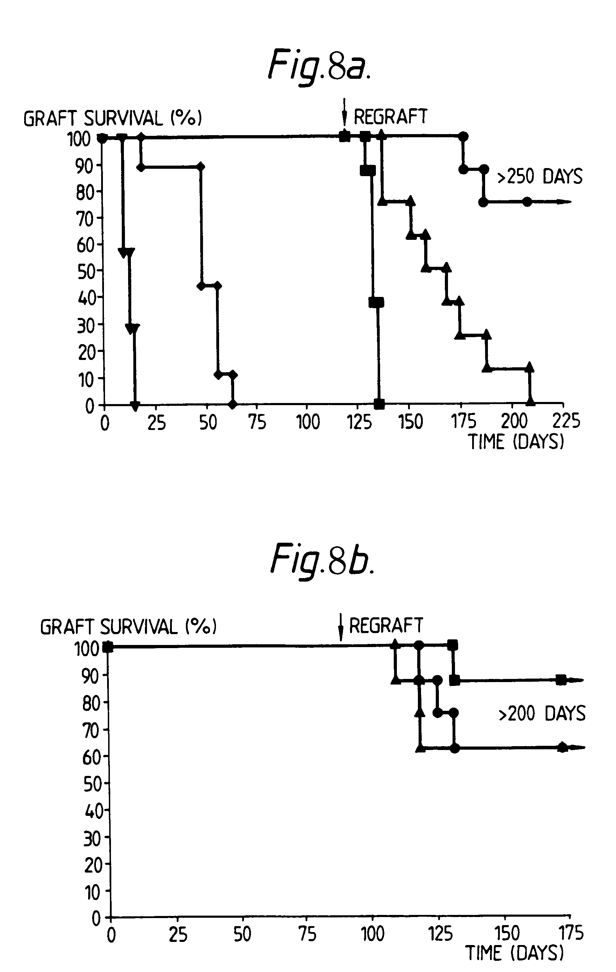

Figure 8 shows the induction of tolerance to MHC-mismatched skin grafts. Groups of 8-12 CBA/Ca mice were grafted with B10 skin on day 0, and treated with monoclonal antibodies from day 0 to 21, as described in Example 2.

(a) The first group were control mice (▼) given no antibody. A second group (♦) were given only rat $IgG_{2a}$ antibodies, while the remaining group (●) all received the combined $IgG_{2b}$ and $IgG_{2a}$ protocol. This last group were given new B10 skin grafts (▲) and third party BALB/c skin (■) on day 119.

Analysis of graft survival: control (no antibody) group MST=14 days compared to $IgG_{2a}$ group MST=48 days P<0.001: Combined protocol original B10 grafts MST>250 days compared to second B10 grafts MST=44 days (from day 119) P<0.002: second B10 grafts compared to third party BALB/c grafts MST=13, P<0.003.

(b) A group of 8 CBA/Ca mice were given B10 skin grafts together with the combined $IgG_{2b}$ followed by $IgG_{2a}$ antibody protocol (●). The mice were given second B10 skin grafts on day 91 (▲) together with third party B10.BR skin (■).

Analysis of graft survival: all groups MST>200 days.

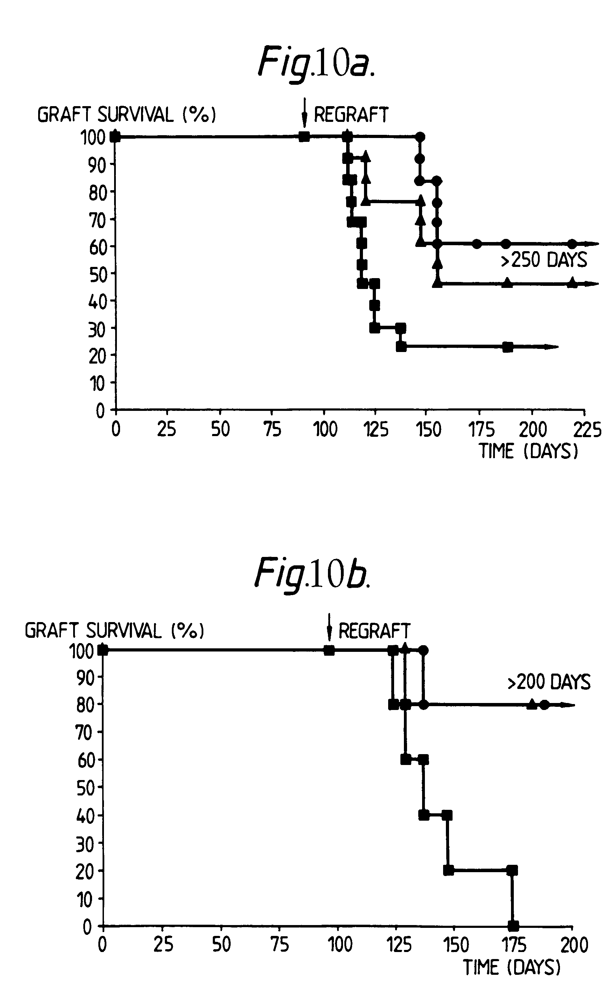

Figure 9 shows the induction of tolerance to multiple minor antigen mismatched skin. Two groups 8 and 10 CBA/Ca mice were given AKR/J skin grafts on day 0. Control mice (▼) received no antibody. The remaining group were given the combined rat $IgG_{2b}$ followed by rat $IgG_{2a}$ antibody protocol (●), and were regrafted at day 122 with fresh AKR/J skin (▲) and third party minors different B10.BR skin (■).

Analysis of graft survival: no antibody controls MST=13 compared to combined protocol original AKR/J grafts MST>250 days, P<0.001: Second AKR/J grafts MST>128 days compared to third party B10.BR grafts MST=27 days, P<0.009.

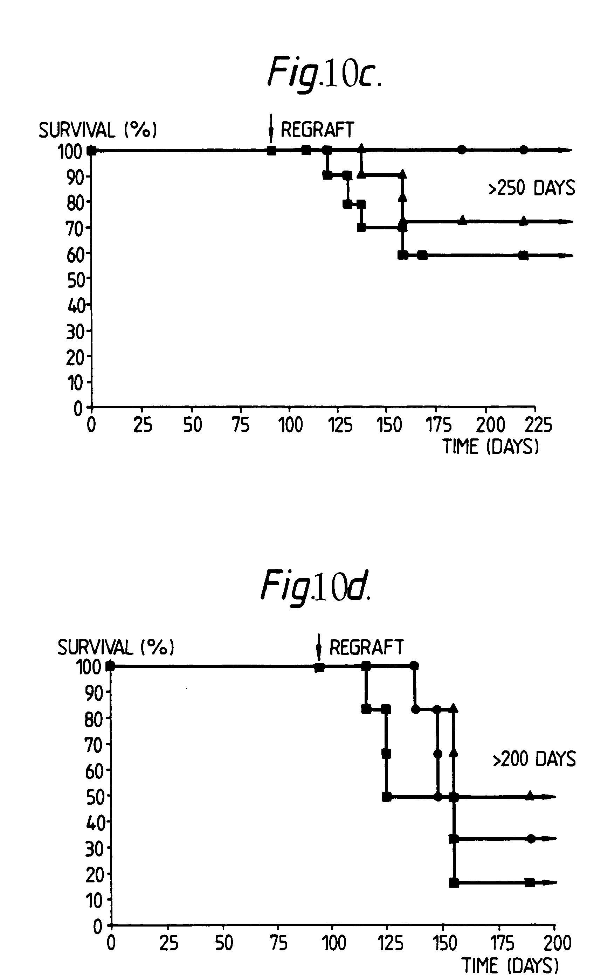

Figure 10 shows the induction of tolerance to multiple minor antigen mismatched skin in primed recipients. Recipient CBA/Ca mice were primed to donor minor antigens by ip injection with $10^7$ irradiated (2000 rad) spleen cells from either AKR/J (a,b) or B10.BR (c,d), three weeks prior to skin grafting. Groups of 5-13 mice were grafted with AKR/J (a,b) or B10.BR (c,d) skin (●) under cover of monoclonal antibodies from day 0-21, as described in Example 2. Two groups received the combined $IgG_{2b}$ and $IgG_{2a}$ protocol (a,c), and two were given rat $IgG_{2a}$ antibodies only (b,d). Mice were regrafted on day 89 (a,c) or day 96 (b,d) with second donor-type (▲) grafts (AKR/J in groups a and b; B10.BR in groups c and d) and third party minors different (■) skin (B10.BR in groups a and b; AKR/J in groups c and d).

Analysis of graft survival: (a) combined protocol original AKR/J grafts MST>225 days compared to third party B10.BR grafts MST=41 days (from day 89), P<0.007. (b)$IgG_{2a}$ treated original AKR/J grafts MST>200 days compared to third party B10.BR grafts MST=42 days (from day 96), P<0.02. (c) all groups MST>day 225 of experiment (d) all groups MST>day 200 of experiment.

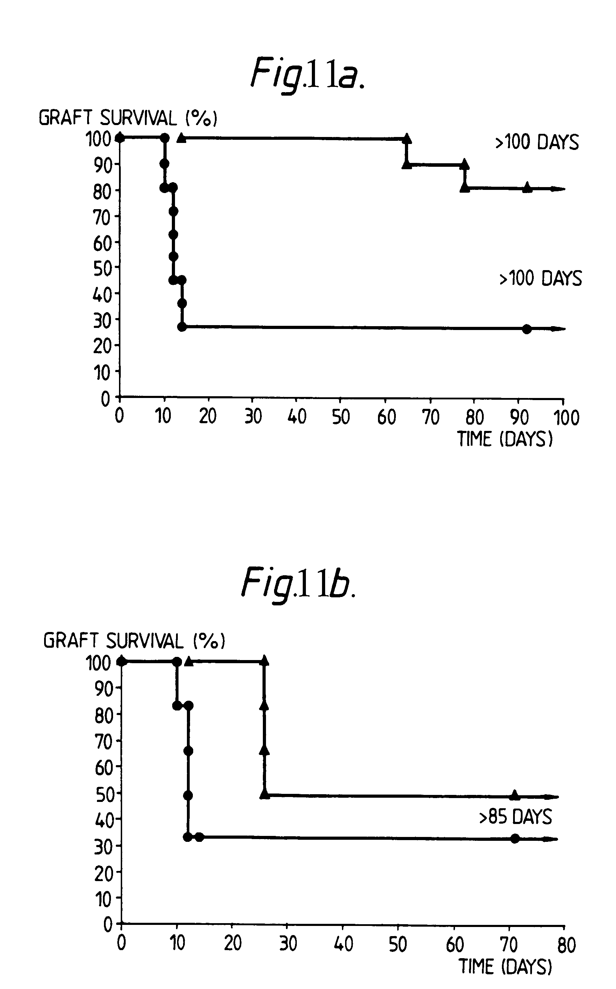

Figure 11 shows the induction of tolerance in mice actively rejecting a first skin graft. Two groups of 6 and 11 CBA/Ca mice were grafted with AKR/J skin on day 0, without any monoclonal antibody treatment. On day 14, when roughly half the mice had rejected these first grafts (●), second AKR/J skin grafts were given in a fresh graft bed and monoclonal antibody treatment initiated (▲). Mice then received either the combined rat $IgG_{2b}$ followed by $IgG_{2a}$ three week protocol (a) or rat $IgG_{2a}$ antibodies alone (b).

Analysis of graft survival: (a) first AKR/J grafts MST=12 days compared to second, combined protocol treated AKR/J grafts MST>92 days, P<0.004. (b) second $IgG_{2a}$ treated AKR/J grafts MST>75 days, compared to all first grafts in (a) and (b) MST=12 days; P<0.003.

## EXAMPLE 1

### MATERIALS AND METHODS

#### Experimental Animals

CBA/Ca, B10.BR and BALB/c mice were bred and kept in the conventional animal facility in the Department of Pathology, University of Cambridge, and were used in age- and sex-matched groups.

#### Identification of Monoclonal Antibodies by Flow Cytometry

A rat T-cell line NB2-6TG (kindly provided by Dr. J. Howard) was transfected with the mouse CD4 gene (Gorman et al, PNAS USA 84, 7644, 1987). This line, NB2.L3T4.Hyg/2.1 was used to screen new CD4 mAbs from a rat anti-mouse thymocyte fusion. Cells ($1-5 \times 10^6$) were incubated with test supernatants, followed by FITC-conjugated rabbit anti-rat Ig serum (Dako, Glostrup, Denmark). The fluorescence staining was analysed by flow cytometry on a Cytofluorograph (model 50-H Ortho, Westwood, MA, USA) and data were processed by an Ortho 2150 computer.

For competitive binding of mAbs, L3T4/Hyg 2.1 cells were incubated firstly with biotinylated mAbs ($1 \mu g/10^5$ cells) on ice in the presence of $10 \mu g$ unlabelled mAbs for 30 minutes. Streptavidin-FITC (Amersham, U.K.) was added for a further incubation of 15 minutes. The results were analysed by the Ortho Cytofluorograph.

Two-colour fluorescence staining was carried out by serially incubating mouse spleen cells with biotinylated first mAb, streptavidin-phycoerythrin (Serotech, Kidlington, U.K.), second mAb and FITC-conjugated, isotype specific mAbs. The green and red fluorescence was detected and displayed on a log scale by the Ortho Cytofluorograph.

#### Immunofluorescence staining of peripheral blood lymphocytes (PBL)

Mouse PBL were separated by centrifugation on Ficoll (specific density 1.079, Phamacia, Sweden) at 3000 x g for 20 minutes. Cells at the interface were collected and washed in PBS/BSA/azide. They were then stained either with $rIgG_{2b}$ mAbs in tissue culture supernatant, followed by FITC conjugated NORIG 7.16 (anti-$rIgG_{2b}$) or with biotinylated mAbs and followed by FITC-streptavidin (Amersham). The results were analysed by flow cytometry as above.

#### Complement lysis

Complement lysis was performed as described previously. In brief, mAbs were diluted and incubated with $^{51}$Cr labelled mouse thymocytes and 2% guinea pig serum added as the complement source. After 45 minutes at 37°C, the supernatant was collected and radioactivity measured by a Philips Automatic Gamma Counter (Philips, Eindhoven, The Netherlands). The specific lysis was calculated as

$$\% \text{ of lysis} = (e-s)/(t-s) \times 100,$$

where e is the sample count, s is the spontaneous release and t is the total count.

#### Induction of Tolerance to Human Gamma Globulin (HGG)

The method of tolerance induction to HGG has been described previously (Benjamin et al, J. Exp. Med 163, 1539, 1986). Normal CBA/Ca mice were injected with CD4 mAbs intravenously (i.v.) on day 1, intraperitoneally (i.p.) on day 0 and 1. lmg of heat-aggregated HGG was given i.p. on day 0. The mice were re-challenged with 0.5mg HGG on days 21 and 31. On day 38, the IgG anti-HGG response was measured by ELISA.

## Detection of Mouse Antibody Responses by ELISA

To measure murine antibody responses, sample sera were serially diluted in flat-bottom flexible plates (Becton Dickinson, USA) coated with purified protein antigens and incubated for 30 minutes. The plates were washed with PBS/0.05% Tween 20 (Sigma, Poole, UK) and then incubated with biotinylated sheep anti-mouse IgG (Amersham) for 30 minutes, and washed as above. Streptavidin-horse radish peroxidase (Amersham) was added in the plates for 15 minutes and after another 3 washes, the reaction was developed by 5mg/ml o-phenolenediamine and 0.1% hydrogen peroxide. The absorbence at 490nm was read and titres determined by comparison with a standard positive control.

## Skin Transplantation

Skin grafts were transplanted by a modified method of Billingham et al (Nature 172, 603, 1953). Recipient mice were anaesthetised with 1µg/mouse Hypnodil and 0.2µg/mouse Sublimase (Janssen, Oxford, UK) via i.p. injection. Full-thickness skin graft from donor tail (0.5cm x 0.5cm) was transplanted onto the graft bed on the lateral thoracic wall, covered with vaseline gauze, cotton bandage and protected by plaster for 7 days. The graft survival was observed and recorded daily thereafter, the end point of survival being the last day when no live graft tissue could be seen.

The median survival time (MST) was calculated and analysed by the Logrank method (Peto et al, Br. J. Cancer 35, 1, 1977). Mice were thymectomised at 4 weeks of age by the method of Monaco et al (J. Immunol, 96, 229, 1966) and used at least 4 weeks later. All were checked at sacrifice and none were found incompletely thymectomised.

## RESULTS

### Identification of rIgG$_{2a}$ mAb Binding to CD4 Molecule

A mAb, YTS 177.9, was isolated on the basis of its binding to an L3T4 transfected cell line using immuno-fluorescence staining (Table 1). Its isotype was determined by reaction with an anti-rIgG$_{2a}$ mAb, RG 7/1.7 (Springer et al, Hybridoma 1,257, 1982) by ELISA (data not shown). Further analyses were carried out to compare this mAb with two other CD4 mAbs described previously. Binding inhibition studies showed that YTS 177.9 bound to the same epitope as YTS 191.1 (Cobbold et al, 1984, Nature 312, 548) and GK 1.5 (Dialyras et al, Imm. Rev. 74, 29-56, 1983) (both CD4 mAbs) but different to that bound by another CD4 mAb YTA 3.1 (Qin et al, Eur. J. Immunol, 17, 1159, 1987) which recognises a separate epitope. In two-colour flow cytometry, YTS 177.9 and YTA 3.1 stained the same population of mouse spleen cells, which is distinct from those stained by a CD8 mAb, YTS 169.4 (Cobbold et al, 1984, Nature 312, 548).

The specificity of YTS 177.9 was also tested by specific $^{51}$-Cr release assay. Although the mAb was inefficient at lysis when used alone, it exhibited synergy with the CD4 mAb YTA 3.1. (Fig. 1a). On the other hand, YTS 177.9 interfered with the lysis mediated by rIgG$_{2b}$ mAb YTS 191.1 which in fluorescence staining was shown to be cross-inhibitory (Fig. 1b).

### The rIg2a CD4 mAb Does Not Deplete T cells in vivo

To determine the effect of the rIgG2a CD4 mAb on in vivo cell depletion, we used adult thymectomised (ATX) mice because in these animals, T-cell recovery following mAb depletion is much less efficient than in normal mice, due to the absence of thymus-dependent T-cell regeneration. By monitoring the change of peripheral T cells after mAb injection, it was possible to distinguish true depletion from temporary antigenic modulation or lymphocyte re-distribution. Each mouse received 2mg of either IgG2a or IgG2b antibodies and peripheral blood lymphocytes were analyzed by flow cytometry before and at various times after treatment. As can be seen in Fig. 2, one week after completion of the IgG2b mAb therapy, about 90% of the CD4$^+$ cells were depleted from the periphery. This was accompanied by a decreased percentage of total T cells and a slight increase of CD8$^+$ cells. In contrast, although the IgG2a mAb treatment reduced the percentage of CD4$^+$ cells, the total percentage of Thy-1$^+$ cells did not change significantly, nor did that of CD8$^+$ cells. This is considered to be the result of antigenic modulation of CD4 molecules following antibody binding. Four weeks after treatment, the T-cell numbers in IgG2b treated groups still remained low, whilst the YTS 177.9 treated group showed a normal level of CD4$^+$ cells.

### Induction of Tolerance to HGG under Cover of rIgG2a CD4 mAb

Previous work using CD4 F(ab')$_2$ mAb fragments has suggested that mAb facilitated tolerance to protein antigens may not need the depletion of CD4$^+$ T-cells. In the present study, we investigated if the non-depleting CD4 mAb, like F(ab')$_2$ fragments, could have the same effect. Mice were given YTS 177.9 or YTS 191.1 in a 3-day course and one injection of HGG (0.5mg/mouse) on the second day (Day 0). When re-challenged with HGG 4 weeks later, YTS 191.1 treated mice and mice given high dose (Img/mouse) of YTS 177.9 had become tolerant to HGG (Fig. 3). However, mice that had received 0.1mg or less of the mAb were still able to respond to HGG.

### The Ability of YTS 177.9 to Induce Tolerance to rIgG2a mAbs

In vivo administration of xenogeneic antibodies usually elicits antiglobulin responses in the host. One of the features of CD4 mAbs is their ability to suppress this antiglobulin response and even to induce tolerance to other antibodies of the same isotype. In the present study, it was found that the rIgG2a CD4 mAb YTS 177.9 had the same property.

Mice given 0.1-1mg/mouse YTS 177.9 made no primary antiglobulin response, (titre: <1:20). However, mice given 0.01 mg/mouse YTS 177.9 did mount a weak response (1:160). Six weeks after the first antibody injection, these animals were rechallenged with 'irrelevant' (rat anti-human) rIgG2a and rIgG2b mAbs, in Freund's adjuvant to ensure adequate stimulation. The rIgG2a was YTH 34.5 which is a mAb to human CDw52 (Waldmann et al, 1985, Adv. Exp. Med. Biol. 186, 869). The rIgG2b was YTH 12.5 which is a mAb to human CD3 (Waldmann et al, 1985). Mice were bled 4 weeks later and their serum anti-rat Ig titres were measured by ELISA. Just as rIgG2b mAbs rendered mice tolerant to rIgG2b immunoglobulins, mice given a high dose of YTS 177.9 (Img) were shown to have become completely tolerant to rIgG2a even after repeated boosts (Fig. 4). 0.1mg YTS 177.9 was insufficient to induce tolerance although it had been immunosuppressive for primary responses. Mice given 0.01mg YTS 177.9 or non-treated control showed secondary-type antiglobulin responses. Tolerance so induced was specific, as YTS 177.9 treated mice were still able to respond to rIgG2b immunoglobulin, while YTS 191.1 tolerant mice could still respond to rIgG2a.

### Delay of Allograft Rejection by rIgG2a mAb Treatment

We found YTS 177.9 as effective as rIgG2b CD4 mAbs in its ability to prolong allogeneic skin graft survival after a short course of treatment. In the first experiment, normal CBA/Ca mice were given daily injections of either rIgG2a (YTS 177.9) or rIgG2b (YTS 191.1) for 2 weeks, starting from 3 days before allogeneic (BALB/c) skin transplantation (approx. 7mg/mouse total mAb). In YTS 177.9 treated group, the MST was significantly prolonged to 20 days (untreated control: 11.3 days; p≤0.05, Table 2). This extended graft survival is very similar to that achieved by rIgG2b CD4 mAb which depleted cells (MST 19 days).

In graft rejection across MHC Class I and II mismatches, the depletion of both CD4$^+$ and CD8$^+$ subsets has been shown to significantly improve the graft survival beyond the effects of CD4 mAbs alone (Cobbold et al, Transplantation, 42, 239, 1986). In the present study, we used another rIgG2a CD8 mAb, which also had little effect on in vivo cell depletion together with the rIgG2a CD4 mAb. This CD8 mAb, YTS 105.18 was found to synergise with the rIgG2a CD4 mAb, YTS 177.9 in further prolonging fully allogeneic skin graft survival. For the purpose of comparison, equal amount of two rIgG2b mAbs (YTS 191.1 and YTS 169.4) or the two rIgG2a mAbs were administered. A total of 3mg/mouse of the mAbs were given on days 0, 2 and 4 relative to skin grafting. The rIgG2b treated CBA/Ca mice rejected BALB/c skin with the MST of 24 days (versus control of 10.5 days, p≤0.005, Fig. 5). Surprisingly, the graft survival in the group treated with rIgG2a CD4 and CD8 mAbs was even longer than that of mice treated with rIgG2b mAbs. 3 out of 4 rIgG2a-treated CBA mice did not reject their BALB/c skin grafts until 45 days after transplantation (MST 46.5 days; versus IgG2b treated group, p≤0.03).

### Tolerance to Skin Allografts

Although there have been reports documenting the permanent survival of certain allografts such as heart or pancreas after CD4 mAb treatment, it has been generally found more difficult to maintain a long-term skin graft with mAb treatment alone. We have previously shown that treatment with CD4 depleting mAbs allowed the acceptance of multiple minor mismatched bone marrow, and such mice were then tolerant of donor skin. In the present study, we grafted CBA/Ca mice with skin from B10.BR mice and treated the recipients with YTS 177.9 (CD4) and YTS 105.18 (CD8). In this H-2 compatible, multi-minor transplantation antigen mismatched combination, all the mice given a 3-week course of mAb treatment accepted the first skin for 90 days. At this time, a second donor-type skin, together with a third party (B10.D2) graft was transplanted. The data in Fig. 6 shows that the first and second grafts all continued to survive indefinitely (>90+ days) while the B10.D2 skin was rejected promptly. Of the mice that had been given only 2 injections of the mAbs, 3 of 6 maintained the B10.BR skin for 90 days but these were slowly rejected after the second skin graft.

EXAMPLE 2

MATERIALS AND METHODS

Mice

CBA/Ca, C57Bl.10/Pc, BALB/c, B10.BR and AKR/J mice were bred and maintained in the conventional animal facility of the Department of Pathology, University of Cambridge. Sex matched groups of mice were used at 6-12 weeks of age.

Monoclonal Antibodies

All the rat monoclonal antibodies used in these experiments are listed in Table 3. Antibodies were prepared from ascitic fluid produced in Pristane-primed (DAxLOU)$F_1$ rats by precipitation with 50% saturated ammonium sulphate followed by dialysis into phosphate buffered saline (PBS;pH7.2). The protein concentration was adjusted to 10mg/ml as estimated by $OD_{280}$ containing 2-5mg/ml of monoclonal antibody. All antibody preparations were passed through a 0.2 micron filter before administration to mice.

Skin Grafting

Skin grafts were performed as previously reported (Cobbold et al, 1986, Transplantation, 41, 634). In brief, donor tail skin grafts (0.5cm to 1.0cm square) were transplanted onto the lateral thoracic wall of recipient mice under anaesthesia. The grafts were covered with gauze and plaster bandage for 7 days. Graft status was documented three times per week for the first month, and approximately weekly thereafter. Differences in survival were analyzed using the Log-Rank method (Peto et al, 1977, Br. J. Cancer, 35, 1). When performing multiple re-grafts from a range of donors, these were transplanted into a single prepared graft bed, usually on the opposite flank to the original skin graft (Cobbold et al, 1986, Nature, 323, 164). Grafts were observed three times per week initially, and weekly thereafter. Median graft survival times (MST) are given as the number of days until rejection is complete, but tolerated grafts with indefinite survival times were generally in good condition with normal hair growth.

Induction of Tolerance to Skin Grafts

Skin grafts were used as the only source of antigen/ tolerogen, and were performed as described above, on the same day as the start of monoclonal antibody treatment. Monoclonal antibodies were administered three times per week from day 0 to day 21 (inclusive), with the first two injections (day 0 and day 2) intra-venous, and the remainder intra-peritoneal. The total dose of all antibodies given per injection was 2mg of ascitic immunoglobulin fraction in 0.2ml of PBS. For rat IgG2b antibodies this was 0.5mg each of YTS 191.1.2, YTA 3.1.2, YTS 169.4.2 and YTS 156.7.7 (a synergistic depleting cocktail of CD4 and CD8 monoclonal antibodies; (Qin et al, 1987, Eur. J. Immunol. 17, 1159).
In the case of rat IgG2a antibodies, Img each of YTS 177.9.6 (CD4) and YTS 105.18.10 (CD8) were used. For the combined depletion and blocking protocol, the synergistic rat IgG2b cocktail was given i.v. on days 0 and 2 and the rat IgG2a CD4 and CD8 three times per week thereafter i.p. until day 21.

Levels of Active Monoclonal Antibody in Serum

Serum samples taken from individual mice at various times during and after monoclonal antibody administration were added, 25μl at 1/5 dilution in PBS containing 1% w/v bovine serum albumin (BSA) plus 5% v/v heat inactivated normal rabbit serum, to $5 \times 10^5$ CD4 or CD8 transfected cells. The CD4 transfectant was the rat NB2-6TG line expressing mouse L3/T4 (Example 1) while the CD8 (Lyt-2) gene was expressed at high levels in mouse L-cells (Zamoyska, 1985, Cell, 43, 153). Bound antibody was detected using monoclonal anti-rat IgG2a coupled to FITC (MARG2A-FITC; Serotec, Oxford, UK) at the manufacturer's recommended dilution, and analysis was on an Ortho 5OH Cytofluorograf with 1250 computer (Ortho, Westwood, MA, USA). The mean fluorescence was compared to a standard dilution series of purified CD4 or CD8 antibody (YTS 177.9.6 or YTS 105.18.10) in normal mouse serum to derive equivalent concentration values for the serum samples. Where the fluorescence showed saturating antibody, or no detectable fluorescence above background, the result was assumed to be greater than or less than the titratable range of the standard curve.

<u>Preparation of Peripheral Blood Leucocytes</u>

Mice were bled individually from the tail vein (approx. 0.1ml) into sterile tubes containing 1U heparin. The plasma was then removed after centrifugation in a microfuge (6000 rpm) for 2 mins. Red blood cells were lysed twice by adding 0.9ml of water for 10 secs at room temperature followed by 0.1ml of 10X phosphate buffered saline and the white cells collected by centrifugation at 1,000 rpm for 7 mins (Chandler <u>et al</u>, 1979, J. Immunol. Methods <u>31</u>, 341).

<u>Monitoring T-cell Subsets for Depletion</u>

Peripheral blood leucocytes were prepared from individual treated mice as described above. These were stained with supernatants containing monoclonal antibodies against either CD4 (YTS 191.1.2 plus YTA 3.1.2) or CD8 (YTS 169.4.2 plus YTS 156.7.7) antigens. Other monoclonal antibodies (see Table 6) were also used as controls. Bound antibody was detected using a mixture of monoclonal anti-rat IgG2b-FITC (NORIG-7.16-FITC; Clark, 1986, Methods Enzymol. <u>121</u>, 548) and monoclonal anti-rat kappa light chain (MAR-18.5-FITC; Lanier <u>et al</u>, 1982, Hybridoma, 1, 125). The fluorescence was analyzed using an Ortho 50H Cytofluorograf with 1250 computer and linear amplifiers, with gating on forward and ninety degree scatters to select for live lymphocytes.

<u>Mixed Lymghocyte Cultures</u>

Responder cells were obtained from individual mouse blood samples (approx 0.2ml) by water lysis as described above. Each sample was assayed for proliferation by splitting the cells into three U-bottom tissue culture microtitre wells (approx $4x10^5$ white cells per well) containing $4x10^5$ well washed, mitomycin-C (Sigma, Poole, UK; $25\mu g/ml$) treated stimulator spleen cells. The final volume of Iscove's modified Dulbecco's medium (IMDM) containing 10% heat inactivated human AB serum was 0.1ml. After 3 days at 37°C in a 5% $CO_2$ gassed incubator, $5\mu l$ of $^{125}$I-deoxyuridine (IUDR; $10\mu Ci/ml$, Amersham, UK) was added for six hours. The $^{125}$I incorporation was measured by harvesting the cells onto glass fibre filters and counting on a Philips Automatic Gamma Counter.

<u>A Combination of Depleting and Blocking CD4 and CD8 Antibodies is Permissive for Tolerance to MHC-Mismatched Skin Grafts</u>

Example 1 has shown that three weeks of therapy with non-depleting CD4 and CD8 antibodies permitted tolerance to multiple minor incompatible skin grafts. In order to establish a treatment protocol that might tolerize across strong MHC differences we compared the effects of administering depleting (rat IgG2b), non-depleting (blocking rat IgG2a) and a combination of depleting followed by blocking CD4 and CD8 antibodies to CBA/Ca (H-2k) mice grafted with BALB/c (H-2d) skin (Figure 7). As we have previously reported, a strictly depleting protocol delayed rejection significantly, but all mice rejected within 70 days (MST = 55 days). Non-depleting antibodies were here less effective (MST = 28 days), but a combination of two depleting doses followed by blockade with rat IgG2a antibodies gave the longest graft survival (MST > 100 days), although most (but not all) grafts were rejected by 200 days. In this experiment, second BALB/c test grafts were given at day 94, together with third party B10 (H-2b) skin. These third party grafts were rejected promptly (MST = 16 days), demonstrating that the mice had returned to immunocompetence, whilst the second BALB/c grafts survived for a median of 43 days. This shows that there must have been some degree of specific unresponsiveness to BALB/c graft antigens.

Although we did not obtain complete tolerance in this strain combination, we found the same combined protocol to be fully tolerance permissive when B10 (H-2b) skin was grafted onto MHC incompatible CBA/Ca (H-2k) mice. Figure 8 shows that 6/8 recipient mice kept their original skin grafts indefinitely (> 250 days), while all mice rejected the third party BALB/c skin grafted on day 119 within 15 days. Second B10 grafts had substantially extended survival times (MST = 44 days), but all were eventually rejected even when the first grafts, assumed to be genetically identical, were maintained. This result is reproducible and shows clearly that the tolerated first graft enjoys privilege of tenure, despite the presence of effector cells capable of rejecting the second graft. Whatever the mechanism, the original skin graft must have induced, and maintained, a state of unresponsiveness to itself. The ability of mice to distinguish between the first and second B10 grafts seemed to be dependant on rejection of the third party skin, as 5/8 mice given B10.BR instead of BALB/c grafts remained tolerant to all three (Figure 8). This demonstrates that the recipients were tolerant of B10 minor antigens in the context of both donor MHC, which means that the graft can itself present for tolerance, and also recipient-type MHC, which must have been through reprocessing of the original graft antigens and presentation for tolerance by recipient APCs.

This is the first report of antibody mediated tolerance across complete MHC barriers using highly immunogenic skin grafts as the only source of tolerogen. It should be stressed that the graft itself must have provided all the antigen presentation necessary for tolerance to occur.

## The Combination of Depleting and Blocking Antibodies Also Permits Tolerance to Skin Matched for MHC but Mismatched for Multiple Non-MHC Minor Antigens

In Example 1 we have demonstrated that blocking with monoclonal antibodies to CD4 and CD8 was sufficient to obtain tolerance to skin grafts differing in multiple minor antigens (B10.BR onto CBA/Ca). In Figure 11 it can be seen that the combined depleting followed by blocking protocol was also effective with 8/10 CBA/Ca mice keeping their AKR/J grafts indefinitely. Six of these mice also held second AKR/J grafts (skin grafted at day 122 without further antibody therapy), while all mice rejected third party minor mismatched skin (B10.BR). The rejection of this third party skin was, however, somewhat slower than normal CBA controls (27 days compared with the normal 14; Cobbold et al, 1986, Transplantation, 41, 634). The reverse combination (B10.BR onto CBA/Ca) was also rendered tolerant using the depletion followed by blocking protocol (4/5 grafts > 220 days), but 2/5 mice accepted both the third party minors difference AKR/J skin as well as the second B10.BR graft (data not shown). This presumably reflects the large number of shared minor antigens and is reminiscent of the finding of "dominant" tolerance seen in a different experimental model (Zamoyska et al, 1989, Eur. J. Immunol. 19, 111).

In summary for this section, we wish to conclude that CD4 and CD8 antibodies, when used appropriately, permit tolerance to be induced across both MHC and non-MHC antigen differences, to skin grafts alone, without the need for any further haemopoietic source of antigen, nor other myeloablative therapy. Clearly, skin grafts are able to present antigen directly to peripheral T-cells either for activation, which leads to rejection, or for inactivation, which instead lead to acceptance and tolerance.

## The Effects of Combined Rat IgG2b and IgG2b Antibodies on Circulating T-cells

In view of the remarkable tolerogenic effects seen in the above experiments, it was important to assess the effects of the monoclonal antibodies on circulating $CD4^+$ and $CD8^+$ T-cells. We have shown previously that rat IgG2b antibodies to either CD4 or CD8 deplete their target T-cells (Cobbold et al, 1984, Nature, 312, 548). In Example 1 rat IgG2a antibodies, in the doses given, tended only to modulate the antigen from the cell surface. In the case of the combined protocol, the initial doses of rat IgG2b CD4 and CD8 monoclonal antibodies caused a reduction in the number of T-cells as expected, and then the proportion of $CD4^+$ and $CD8^+$ cells actually started to recover during the continued treatment with IgG2a antibodies (Table 4), although the amount of antigen expressed was much reduced (data not shown). Residual antibody was present on the T-cells throughout the administration period, making detailed measurements of $CD4^+$ and $CD8^+$ cells difficult.

Thereafter, the proportion of T-cells in the peripheral blood remained diminished for at least one month after stopping the antibody, an effect not observed when IgG2a antibodies were given alone in equivalent does (data not shown).

One mechanism for the suppression of graft rejection by non-depleting monoclonal antibodies is through blocking the function of CD4 and CD8 accessory molecules on the T-cell surface during antigen presentation. This would be most effective only if serum antibody was maintained at levels sufficient to saturate antigen positive cells. The levels of active antibody in treated mice was indeed found to be sufficient to saturate the target CD4 and CD8 antigens throughout the three weeks of treatment, and in some mice up to three weeks after stopping antibody administration (Table 5). However, by day 60, there was no detectable (< 0.5ng/ml CD4 and < 10ng/m1 CD8) monoclonal antibody left in the serum which could otherwise have maintained a nonspecific immunosuppression. It should be noted that none of the mice made any detectable antiglobulin (neither anti-species nor anti-idiotype) as measured by a capture ELISA, indicating that mice were also rendered tolerant of rat immunoglobulin by this protocol.

## Tolerant Mice Can Still Respond In Vitro

We found that CBA mice rendered tolerant of B10 skin as described above were still capable of proliferating in vitro to B10 stimulator spleen cells (Table 6), showing that a significant number of allo-reactive T-cells had not been deleted. Clearly the recipients immune system is tolerant to the antigens presented by the original graft, but is still capable of reacting to in vitro presentation by spleen stimulator cells or a second skin graft.

## The Induction of Tolerance in Mice Previously Primed To Minor Antigens

Given that virgin T-cells in the peripheral immune system could be tolerized by graft-born antigens, we sought to determine if primed T-cells were also tolerance susceptible. We found that both of the two treatment protocols (blocking alone, or depletion followed by blocking) were effective for the induction of tolerance to either AKR/J or B10.BR skin in CBA/Ca mice previously primed with irradiated donor spleen cells (Figures 10a-10d). In contrast, we have previously shown that depleting the $CD4^+$ and $CD8^+$ T-cells, although equally immunosuppressive in naive or primed mice, was not tolerance permissive for multiple minor antigen mismatched skin, as all the grafts were eventually rejected (Cobbold et al, 1986, Transplantation, 41, 634).

The only difference between the naive mice of Figure 9 and the primed mice of Figure 10 was seen after rechallenge. About half the mice in the group which had been primed and tolerized to AKR/J eventually rejected both first and second AKR/J grafts, although more slowly than the B10.BR third party grafts (MST = 63 and 21 days respectively; Figure 10a). In the B10.BR onto CBA combination the majority of mice held the original grafts indefinitely, while, in some mice, both the second B10.BR and AKR/J grafts rejected slowly (Figure 10c). This difference in behaviour of the strain combinations is compatible with that seen in naive mice (see Figure 9) and probably lies in the complex pattern of shared and unique minor antigens.

## Tolerance Induction During Active Rejection of a Skin Graft

The finding that even sensitized T-cells are tolerizable has obvious clinical implications. It would clearly be advantageous to induce tolerance in the face of an ongoing immune response, especially in autoimmunity or during an organ rejection crisis after transplantation. In Figure 11 it can be seen that it is indeed possible to reverse ongoing rejection of multiple minor antigen mismatched skin grafts and establish long-term survival, in some cases in both the first and second grafts. In this experiment, combined depletion followed by blockade was most effective (Figure 11a), but as 3/6 of the mice given the blocking (non-depleting) antibodies also held their second grafts (Figure 11b), it must be possible even for effector T-cells to be rendered inactive or tolerant.

TABLE 1

| Binding of YTS 177.9 on L3T4 transfected cells | |
|---|---|
| Mab | % of positive cells L3T4[a] |
| YTS 177.9[b] | 98.7 |
| YTS 191.1[b] | 99.1 |
| YTS 177.9[c] and YTS 191.1 | 1.1 |
| YTS 177.9[c] and YTA 3.1 | 98.1 |

a L3/T4 transfected cell lines stained with respective Mabs and analysed by flow cytometry.

b Mabs were used as tissue culture supernatant followed by FITC conjugated rabbit anti-rat Ig.

c Biotinylated Mabs, which were incubated with excess amount of 'cold' Mabs given underneath. The fluorescence was developed with FITC-strepavidin.

## Table 2 Delay of skin graft rejection by IgG2a and IgG2b CD4 Mabs

| MAB TREATMENT[a] | % OF CELLS[b] | | | GRAFT SURVIVAL (DAYS) | |
|---|---|---|---|---|---|
| | CD4$^+$ | CD8$^+$ | Thy-1$^+$ | B10.BR | BALB/c |
| YTS 191.1 | 1.9±1.5 | 17.3±1.7 | 20.2±4.4 | 28,29,32, 58,65. | |
| YTS 191.1 | 1.2±1.1 | 18.1±2.3 | 24.5±3.6 | | 15,18,19, 19,20,21. |
| YTS 177.9 | 28.3±9.8 | 15.3±0.4 | 48.8±5.0 | 15,20,22,>120, >120,>120. | |
| YTS 177.9 | 27.5±10.1 | 16.1±1.2 | 46.9±4.2 | | 19,19,20, 20,21,21. |
| none | 39.8±3.0 | 12.5±1.5 | 47.0±2.8 | 10,11,12 12,13. | 9,10,10, 11,12,12. |

a) Normal CBA/Ca mice were given CD4 Mabs for 2 weeks (appr. 7 mg/mouse) starting from 3 days before skin grafting.

b) Peripheral blood collected 4 days after the last Mab injection were stained with biotinylated YTA 3.1 (CD4), YTS 156.7 (CD8) and YTS 154.7 (Thy-1), followed by streptavidir FITC. The results were analysed by flow cytometry.

c) MST of B10.BR grafts: non-treated control: 12 days; YTS 191.1 treated: 32 days (v.s. control: $p \leq 0.005$).

YTS 177 treated: 22 days (v.s. control $p \leq 0.004$, v.s YTS 191.1 group, $p \leq 0.7$).

MST of BALB/c grafts: non-treated control: 10.5 days; YTS 191.1 treated 19 days (v.s. control, $p \leq 0.006$);

YTS 177.9 treated: 20 days (v.s. control $p \leq 0.006$, v.s. YTS 191.1 group, $p \leq 0.4$).

EP 0 474 691 B1

TABLE 3:

| Monoclonal antibodies used | | | | |
|---|---|---|---|---|
| Monoclonal antibody | Specificity | Epitope | Isotype | Reference |
| YTS 191.1.2 | mouse CD4 | a | IgG$_{2b}$ | Cobbold et al, 1984, Nature, 312, 548 |
| YTA 3.1.2 | mouse CD4 | b | IgG$_{2b}$ | Qin et al, Eur J. Immunol. 17, 1159, 1987 |
| YTS 177.9.6 | mouse CD4 | a | IgG$_{2a}$ | |
| YTS 169.4.2 | mouse CD8 | Lyt-2 (a) | IgG$_{2b}$ | Cobbold et al, 1984, Nature, 312, 548 |
| YTS 156.7.7 | mouse CD8 | Lyt-3 (b) | IgG$_{2b}$ | Qin et al, 1989 J.Exp.Med. 169, 779 |
| YTS 105.18.10 | mouse CD8 | Lyt-2 (c) | IgG$_{2a}$ | |
| YTS 154.7.7 | mouse Thy-1 | monomorphic | IgG$_{2b}$ | Cobbold et al, 1984, Nature, 312, 548 |
| YTS 121.5.2 | mouse CD5 | Lyt-1 | IgG$_{2b}$ | Cobbold et al, 1984, Nature, 312, 548 |

TABLE 4:

| Remaining T-cells in CD4+CD8 antibody treated mice | | | | | |
|---|---|---|---|---|---|
| B10 tolerant CBA Per-cent of spleen cells staining positive | | | | | |
| Time (days) | CD4+ | CD8+ | Thy-1+CD5+ | anti-rat Ig* | number tested |
| 0 | 34±2 | 16±1 | 47±1 | 0±0 | 4 |
| 5 | 12±3 | 12±5 | 13±4 | 6±1 | 8 |
| 12 | 18±4 | 18±6 | 18±5 | 3±1 | 3 |
| 19 | 27±4 | 22±3 | 26±5 | 3±3 | 6 |
| 45 | 24±5 | 11±2 | 28±4 | 0±0 | 8 |

*Anti-rat Ig was a mixture of MAR-18.5-FITC and NORIG-7.16-FITC.

All mice received rat IgG$_{2b}$ CD4 and CD8 followed by rat IgG$_{2a}$ CD4 and CD8 as described in Example 2 from day 0-21.

TABLE 5:

| Serum levels of active IgG$_{2a}$ CD4 and CD8 monoclonal antibodies | | | | | | | |
|---|---|---|---|---|---|---|---|
| CD4 serum antibody (ng/ml) [Four individual mice ] | | | | CD8 serum antibody (ng/ml) [Four individual mice] | | | |
| Time | | | | | | | |
| Day 0 | <0.5 | <0.5 | <0.5 | <0.5 | <10 | <10 | <10 | <10 |
| Day 12 | >100 | >100 | >100 | >100 | >100 | 120 | 150 | >1000 |
| Day 19 | 50 | >100 | >100 | >100 | 190 | >1000 | >1000 | >1000 |
| Day 29 | 50 | >100 | >100 | >100 | 75 | 250 | >1000 | >1000 |
| Day 32 | 80 | 90 | >100 | >100 | 500 | 600 | >1000 | >1000 |
| Day 36 | 5 | 100 | >100 | >100 | 60 | 85 | 500 | >1000 |
| Day 43 | <0.5 | 15 | 70 | >100 | <10 | <10 | 12 | 25 |
| Day 46 | <0.5 | <0.5 | <0.5 | 1.0 | <10 | <10 | <10 | <10 |
| Day 60 | <0.5 | <0.5 | <0.5 | <0.5 | <10 | <10 | <10 | <10 |
| Day78 | <0.5 | <0.5 | <0.5 | <0.5 | <10 | <10 | <10 | <10 |

Monoclonal antibody treatment was rat IgG$_{2b}$ CD4 and CD8 on days 0 and 2, followed by rat IgG$_{2a}$ CD4 and CD8 three times per week until day 21 (2mg total per injection).

TABLE 6:

| Proliferation of Peripheral Blood Lymphocytes from B10 tolerant CBA mice | | |
|---|---|---|
| Proliferation ($^{125}$IUDR incorporation) to stimulators | | |
| Group | CBA (cpm.) | BALB/c (cpm.) | B10 (cpm.) |
| Normal CBA control | 370+236 -121 | 4952+5236 -2624 | 2210+2622 -1285 |
| B10 tolerant CBA | 716+800 -448 | 4952+3470 -2091 | 3592+2875 -1656 |

Individual B10 tolerant CBA/Ca mice, or normal controls, were bled from the tail vein on day 78. Proliferation of peripheral blood cells was determined as in Example 2.

Figures given are logarithmic means and standard deviations of six mice per group.

## Claims

**Claims for the following Contracting States : AT, BE, CH, LI, DE, DK, FR, GB, IT, LU, NL, SE**

1.  Use of a non-depleting anti-CD4 monoc onal antibody, i.e. an antibody which causes depletion of fewer than 50% of CD4+ T-cells from the periphery as measured by changes in the peripheral blood lymphocyte numbers, for the manufacture of a medicament for the induction of a state of immunological tolerance to an antigen by a method which comprises administering said non-depleting anti-CD4 monoclonal antibody to a subject together with a non-depleting anti-CD8 monoclonal antibody, i.e. an antibody which causes depletion of fewer than 50% of CD8+ T-cells from the periphery as measured by changes in the peripheral blood lymphocyte numbers, to induce an immunological tolerance permissive environment within said subject by means of said antibodies in the presence of said antigen.

2.  Use of a non-depleting anti-CD8 monoclonal antibody, i.e. an antibody which causes depletion of fewer than 50% of CD8+ T-cells from the periphery as measured by changes in the peripheral blood lymphocyte numbers, for the manufacture of a medicament for the induction of a state of immunological tolerance to an antigen by a method which comprises administering said non-depleting anti-CD8 monoclonal antibody to a subject together with a non-depleting anti-CD4 monoclonal antibody, i.e. an antibody which causes depletion of fewer than 50% of CD4+ T-cells from the periphery as measured by changes in the peripheral blood lymphocyte numbers, to induce a tolerance permissive environment within said subject by means of said antibodies in the presence of said antigen.

3.  Use according to claim 1 or 2 characterised in that said antigen is selected from transplantation antigens, autoantigens and immunoglobulins.

4.  Use of a non-depleting anti-CD4 monoclonal antibody, i.e. an antibody which causes depletion of fewer than 50% of CD4+ T-cells from the periphery as measured by changes in the peripheral blood lymphocyte numbers, as the whole antibody for the manufacture of a medicament for the induction of a state of immunological tolerance to an antigen selected from autoantigens, transplantation antigens and immunoglobulins by a method which comprises administering said non-depleting anti-CD4 monoclonal antibody to a subject to induce an immunological tolerance permissive environment within said subject by means of said antibody in the presence of said antigen.

5.  Use according to any of claims 1 to 4 characterised in that said method comprises administering said antibody or antibodies together with one or more immunosuppressive therapies.

6.  Use according to any of claims 1 to 4 characterised in that said method comprises administering a depleting anti-CD4 monoclonal antibody and/or a depleting anti-CD8 monoclonal antibody prior to administration of said non-depleting antibody or antibodies.

7. Use according to any of claims 1 to 6 wherein the non-depleting anti-CD4 antibody and, if used, the non-depleting anti-CD8 antibody are each recombinant antibodies.

8. Use according to any of claims 1 to 7 wherein the antigen is an autoantigen.

9. Use according to claim 8 wherein the medicament is for the induction of tolerance in the treatment of rheumatoid arthritis or multiple sclerosis.

10. Use according to any of claims 1 to 9 wherein the non-depleting anti-CD4 antibody and, if used, the non-depleting anti-CD8 antibody are each administered at a dose of 1 to 400 mg, 1 to 7 times per week.

11. A pharmaceutical composition comprising from 1 to 400 mg per dose of a non-depleting anti-CD4 antibody, i.e. an antibody which causes depletion of fewer than 50% of CD4+ T-cells from the periphery as measured by changes in the peripheral blood lymphocyte numbers, or a non-depleting anti-CD8 antibody, i.e. an antibody which causes depletion of fewer than 50% of CD8+ T-cells from the periphery as measured by changes in the peripheral blood lymphocyte numbers, together with a pharmaceutically acceptable carrier or diluent in a form suitable for administration to a human subject.

12. A pharmaceutical composition according to claim 11 where the dose is from 3 to 30 mg of the antibody.

13. A pharmaceutical composition according to claim 12 where the dose is from 5 to 20 mg of the antibody.

14. A pharmaceutical composition according to claim 11 where the dose is from 20 to 400 mg of the antibody.

15. A pharmaceutical composition according to claim 14 where the dose is from 30 to 400 mg of the antibody.

16. A pharmaceutical composition according to any of claims 11 to 15 wherein the antibody is an anti-CD4 antibody.

17. A pharmaceutical composition according to any of claims 11 to 15 wherein the antibody is an anti-CD8 antibody.


**Claims for the following Contracting State : ES**

1. Use of a non-depleting anti-CD4 monoclonal antibody, i.e. an antibody which causes depletion of fewer than 50% of CD4+ T-cells from the periphery as measured by changes in the peripheral blood lymphocyte numbers, for the manufacture of a medicament for the induction of a state of immunological tolerance to an antigen by a method which comprises administering said non-depleting anti-CD4 monoclonal antibody to a subject together with a non-depleting anti-CD8 monoclonal antibody, i.e. an antibody which causes depletion of fewer than 50% of CD8+ T-cells from the periphery as measured by changes in the peripheral blood lymphocyte numbers, to induce an immunological tolerance permissive environment within said subject by means of said antibodies in the presence of said antigen.

2. Use of a non-depleting anti-CD8 monoclonal antibody, i.e. an antibody which causes depletion of fewer than 50% of CD8+ T-cells from the periphery as measured by changes in the peripheral blood lymphocyte numbers, for the manufacture of a medicament for the induction of a state of immunological tolerance to an antigen by a method which comprises administering said non-depleting anti-CD8 monoclonal antibody to a subject together with a non-depleting anti-CD4 monoclonal antibody, i.e. an antibody which causes depletion of fewer than 50% of CD4+ T-cells from the periphery as measured by changes in the peripheral blood lymphocyte numbers, to induce a tolerance permissive environment within said subject by means of said antibodies in the presence of said antigen.

3. Use according to claim 1 or 2 characterised in that said antigen is selected from transplantation antigens, autoantigens and immunoglobulins.

4. Use of a non-depleting anti-CD4 monoclonal antibody, i.e. an antibody which causes depletion of fewer than 50% of CD4+ T-cells from the periphery as measured by changes in the peripheral blood lymphocyte numbers, as the whole antibody for the manufacture of a medicament for the induction of a state of immunological tolerance to an antigen selected from autoantigens, transplantation antigens and immunoglobulins by a method which comprises administering said non-depleting anti-CD4 monoclonal antibody to a subject to induce an immunological tolerance permissive environment within said subject by means of said antibody in the presence of said antigen.

5. Use according to any of claims 1 to 4 characterised in that said method comprises administering said antibody or antibodies together with one or more immunosuppressive therapies.

6. Use according to any of claims 1 to 4 characterised in that said method comprises administering a depleting anti-CD4 monoclonal antibody and/or a depleting anti-CD8 monoclonal antibody prior to administration of said non-depleting antibody or antibodies.

7. Use according to any of claims 1 to 6 wherein the non-depleting anti-CD4 antibody and, if used, the non-depleting anti-CD8 antibody are each recombinant antibodies.

8. Use according to any of claims 1 to 7 wherein the antigen is an autoantigen.

9. Use according to claim 8 wherein the medicament is for the induction of tolerance in the treatment of rheumatoid arthritis or multiple sclerosis.

10. Use according to any of claims 1 to 9 wherein the non-depleting anti-CD4 antibody and, if used, the non-depleting anti-CD8 antibody are each administered at a dose of 1 to 400 mg, 1 to 7 times per week.

11. A process for the production of a pharmaceutical composition which comprises processing a non-depleting anti-CD4 antibody, i.e. an antibody which causes depletion of fewer than 50% of CD4+ T-cells from the periphery as measured by changes in the peripheral blood lymphocyte numbers, or a non-depleting anti-CD8 antibody, i.e. an antibody which causes depletion of fewer than 50% of CD8+ T-cells from the periphery as measured by changes in the peripheral blood lymphocyte numbers, together with a pharmaceutically acceptable carrier or diluent to form a composition suitable for administration to a human subject and comprising from 1 to 400 mg per dose of the antibody.

12. A process according to claim 11 where the dose is from 3 to 30 mg of the antibody.

13. A process according to claim 12 where the dose is from 5 to 20 mg of the antibody.

14. A process according to claim 11 where the dose is from 20 to 400 mg of the antibody.

15. A process according to claim 14 where the dose is from 30 to 400 mg of the antibody.

16. A process according to any of claims 11 to 15 wherein the antibody is an anti-CD4 antibody.

17. A process according to any of claims 11 to 15 wherein the antibody is an anti-CD8 antibody.

**Patentansprüche**

**Patentansprüche für folgende Vertragstaaten : AT, BE, CH, LI, DE, DK, FR, GB, IT, LU, NL, SE**

1. Verwendung eines nichtdepletierenden monoclonalen Anti-CD4-Antikörpers, d.h. eines Antikörpers, der eine Depletion von weniger als 50 % der CD4+-T-Zellen von der Peripherie, gemessen durch Veränderungen der Anzahlen der peripheren Blutlymphocyten, verursacht, zur Herstellung eines Medikaments zur Induktion eines Zustands immunologischer Toleranz gegenüber einem Antigen nach einem Verfahren, bei dem man den nichtdepletierenden monoclonalen Anti-CD4-Antikörper einem Patienten zusammen mit einem nichtdepletierenden monoclonalen Anti-CD8-Antikörper, d.h. einem Antikörper, der eine Depletion von weniger als 50 % der CD8+-T-Zellen von der Peripherie, gemessen durch Veränderungen der Anzahlen der peripheren Blutlymphocyten, verursacht, verabreicht, um eine für eine immunologische Toleranz permissive Umgebung in dem Patienten durch die Antikörper in Gegenwart des Antigens hervorzurufen.

2. Verwendung eines nichtdepletierenden monoclonalen Anti-CD8-Antikörpers, d.h. eines Antikörpers, der eine Depletion von weniger als 50 % der CD8+-T-Zellen von der Peripherie, gemessen durch Veränderungen der Anzahlen der peripheren Blutlymphocyten, verursacht, zur Herstellung eines Medikaments zur Induktion eines Zustands immunologischer Toleranz gegenüber einem Antigen nach einem Verfahren, bei dem man den nichtdepletierenden monoclonalen Anti-CD8-Antikörper einem Patienten zusammen mit einem nichtdepletierenden monoclonalen Anti-CD4-Antikörper, d.h. einem Antikörper, der eine Depletion von weniger als 50 % der CD4+-T-Zellen von der Peripherie, gemessen durch Veränderungen der Anzahlen der peripheren Blutlymphocyten, verursacht, verabreicht, um eine für eine immunologische Toleranz permissive Umgebung in dem Patienten durch die Antikörper in Gegenwart des Antigens hervorzurufen.

3. Verwendung nach Anspruch 1 oder 2, dadurch **gekennzeichnet,** daß das Antigen aus Transplantationsantigenen, Autoantigenen und Immunglobulinen ausgewählt wird.

4. Verwendung eines nichtdepletierenden monoclonalen Anti-CD4-Antikörpers, d.h. eines Antikörpers, der eine Depletion von weniger als 50 % der CD4+-T-Zellen von der Peripherie, gemessen durch Veränderungen in den Anzahlen der peripheren Blutlymphocyten, verursacht, als ganzen Antikörper zur Herstellung eines Medikaments zur Induktion eines Zustands immunologischer Toleranz gegenüber einem Antigen, ausgewählt aus Autoantigenen, Transplantationsantigenen und Immunglobulinen, nach einem Verfahren, bei dem man den nichtdepletierenden monoclonalen Anti-CD4-Antikörper einem Patienten verabreicht, um eine für eine immunologische Toleranz permissive Umgebung in dem Patienten durch den Antikörper in Gegenwart des Antigens hervorzurufen.

5. Verwendung nach einem der Ansprüche 1 bis 4, dadurch **gekennzeichnet,** daß man bei dem Verfahren den Antikörper oder die Antikörper zusammen mit einer oder mehreren immunsuppressiven Therapien verabreicht.

6. Verwendung nach einem der Ansprüche 1 bis 4, dadurch **gekennzeichnet,** daß man bei dem Verfahren einen depletierenden monoclonalen Anti-CD4-Antikörper und/oder einen depletierenden monoclonalen Anti-CD8-Antikörper vor der Verabreichung des/der nichtdepletierenden Antikörpers/Antikörper verabreicht.

7. Verwendung nach einem der Ansprüche 1 bis 6, wobei der nichtdepletierende Anti-CD4-Antikörper und, sofern verwendet, der nichtdepletierende Anti-CD8-Antikörper jeweils rekombinante Antikörper sind.

8. Verwendung nach einem der Ansprüche 1 bis 7, wobei das Antigen ein Autoantigen ist.

9. Verwendung nach Anspruch 8, wobei das Medikament der Induktion von Toleranz bei der Behandlung der rheumatoiden Arthritis oder multiplen Sklerose dient.

10. Verwendung nach einem der Ansprüche 1 bis 9, wobei der nichtdepletierende Anti-CD4-Antikörper und, sofern verwendet, der nichtdepletierende Anti-CD8-Antikörper jeweils in einer Dosis von 1 bis 400 mg 1- bis 7mal pro Woche verabreicht werden.

11. Pharmazeutische Zusammensetzung, umfassend 1 bis 400 mg pro Dosis eines nichtdepletierenden Anti-CD4-Antikörpers, d.h. eines Antikörpers, der eine Depletion von weniger als 50 % der CD4+-T-Zellen von der Peripherie, gemessen durch Veränderungen in den Anzahlen der peripheren Blut lymphocyten, verursacht, oder eines nichtdepletierenden Anti-CD8-Antikörpers, d.h. eines Antikörpers, der eine Depletion von weniger als 50 % der CD8+-T-Zellen von der Peripherie, gemessen durch Veränderungen in den Anzahlen der peripheren Blutlymphocyten, verursacht, zusammen mit einem pharmazeutisch verträglichen Träger oder Verdünnungsmittel in einer für die Verabreichung an einen Menschen geeigneten Form.

12. Pharmazeutische Zusammensetzung nach Anspruch 11, wobei die Dosis 3 bis 30 mg des Antikörpers beträgt.

13. Pharmazeutische Zusammensetzung nach Anspruch 12, wobei die Dosis 5 bis 20 mg des Antikörpers beträgt.

14. Pharmazeutische Zusammensetzung nach Anspruch 11, wobei die Dosis 20 bis 400 mg des Antikörpers beträgt.

15. Pharmazeutische Zusammensetzung nach Anspruch 14, wobei die Dosis 30 bis 400 mg des Antikörpers beträgt.

16. Pharmazeutische Zusammensetzung nach einem der Ansprüche 11 bis 15, wobei der Antikörper ein Anti-CD4-Antikörper ist.

17. Pharmazeutische Zusammensetzung nach einem der Ansprüche 11 bis 15, wobei der Antikörper ein Anti-CD8-An-

tikörper ist.

**Patentansprüche für folgenden Vertragsstaat : ES**

1. Verwendung eines nichtdepletierenden monoclonalen Anti-CD4-Antikörpers, d.h. eines Antikörpers, der eine Depletion von weniger als 50 % der CD4+-T-Zellen von der Peripherie, gemessen durch Veränderungen der Anzahlen der peripheren Blutlymphocyten, verursacht, zur Herstellung eines Medikaments zur Induktion eines Zustands immunologischer Toleranz gegenüber einem Antigen nach einem Verfahren, bei dem man den nichtdepletierenden monoclonalen Anti-CD4-Antikörper einem Patienten zusammen mit einem nichtdepletierenden monoclonalen Anti-CD8-Antikörper, d.h. einem Antikörper, der eine Depletion von weniger als 50 % der CD8+-T-Zellen aus der Peripherie, gemessen durch Veränderungen der Anzahlen der peripheren Blutlymphocyten, verursacht, verabreicht, um eine für eine immunologische Toleranz permissive Umgebung in dem Patienten durch die Antikörper in Gegenwart des Antigens hervorzurufen.

2. Verwendung eines nichtdepletierenden monoclonalen Anti-CD8-Antikörpers, d.h. eines Antikörpers, der eine Depletion von weniger als 50 % der CD8+-T-Zellen von der Peripherie, gemessen durch Veränderungen der Anzahlen der peripheren Blutlymphocyten, verursacht, zur Herstellung eines Medikaments zur Induktion eines Zustands immunologischer Toleranz gegenüber einem Antigen nach einem Verfahren, bei dem man den nichtdepletierenden monoclonalen Anti-CD8-Antikörper einem Patienten zusammen mit einem nichtdepletierenden monoclonalen Anti-CD4-Antikörper, d.h. einem Antikörper, der eine Depletion von weniger als 50 % der CD4+-T-Zellen aus der Peripherie, gemessen durch Veränderungen der Anzahlen der peripheren Blutlymphocyten, verursacht, verabreicht, um eine für eine immunologische Toleranz permissive Umgebung in dem Patienten durch die Antikörper in Gegenwart des Antigens hervorzurufen.

3. Verwendung nach Anspruch 1 oder 2, dadurch **gekennzeichnet,** daß das Antigen aus Transplantationsantigenen, Autoantigenen und Immunglobulinen ausgewählt wird.

4. Verwendung eines nichtdepletierenden monoclonalen Anti-CD4-Antikörpers, d.h. eines Antikörpers, der eine Depletion von weniger als 50 % der CD4+-T-Zellen von der Peripherie, gemessen durch Veränderungen in den Anzahlen der peripheren Blutlymphocyten, verursacht, als ganzen Antikörper zur Herstellung eines Medikaments zur Induktion eines Zustands immunologischer Toleranz gegenüber einem Antigen, ausgewählt aus Autoantigenen, Transplantationsantigenen und Immunglobulinen, nach einem Verfahren, bei dem man den nichtddepletierenden monoclonalen Anti-CD4-Antikörper an Patienten verabreicht, um eine für eine immunologische Toleranz permissive Umgebung in dem Patienten durch den Antikörper in Gegenwart des Antigens hervorzurufen.

5. Verwendung nach einem der Ansprüche 1 bis 4, dadurch **gekennzeichnet,** daß man bei dem Verfahren den Antikörper oder die Antikörper zusammen mit einer oder mehreren immunsuppressiven Therapien verabreicht.

6. Verwendung nach einem der Ansprüche 1 bis 4, dadurch **gekennzeichnet,** daß man bei dem Verfahren einen depletierenden monoclonalen Anti-CD4-Antikörper und/oder einen depletierenden monoclonalen Anti-CD8-Antikörper vor der Verabreichung des/der nichtdepletierenden Antikörpers/Antikörper verabreicht.

7. Verwendung nach einem der Ansprüche 1 bis 6, wobei der nichtdepletierende Anti-CD4-Antikörper und, sofern verwendet, der nichtdepletierende Anti-CD8-Antikörper jeweils rekombinante Antikörper sind.

8. Verwendung nach einem der Ansprüche 1 bis 7, wobei das Antigen ein Autoantigen ist.

9. Verwendung nach Anspruch 8, wobei das Medikament der Induktion von Toleranz bei der Behandlung der rheumatoiden Arthritis oder multiplen Sklerose dient.

10. Verwendung nach einem der Ansprüche 1 bis 9, wobei der nichtdepletierende Anti-CD4-Antikörper und, sofern verwendet, der nichtdepletierende Anti-CD8-Antikörper jeweils in einer Dosis von 1 bis 400 mg 1- bis 7mal pro Woche verabreicht werden.

11. Verfahren zur Herstellung einer pharmazeutischen Zusammensetzung, wobei man einen nichtdepletierenden Anti-CD4-Antikörper, d.h. einen Antikörper, der eine Depletion von weniger als 5 % der CD4+-T-Zellen von der Peripherie, gemessen durch Veränderungen der Anzahlen der peripheren Blutlymphocyten, hervorruft, oder einen nichtdepletierenden Anti-CD8-Antikörper, d.h. einen Antikörper, der eine Depletion von weniger als 50 % der CD8+-T-Zellen von der Peripherie, gemessen durch Veränderungen in den Anzahlen der peripheren Blutlymphocyten, verursacht, zusammen mit einem pharmazeutisch verträglichen Träger oder Verdünnungsmittel verarbeitet, wobei eine Zusammensetzung gebildet wird, die für die Verabreichung an einen Menschen geeignet ist und 1 bis 400 mg pro Dosis des Antikörpers umfaßt.

**12.** Verfahren nach Anspruch 11, wobei die Dosis 3 bis 30 mg des Antikörpers beträgt.

**13.** Verfahren nach Anspruch 12, wobei die Dosis 5 bis 20 mg des Antikörpers beträgt.

**14.** Verfahren nach Anspruch 11, wobei die Dosis 20 bis 400 mg des Antikörpers beträgt.

**15.** Verfahren nach Anspruch 14, wobei die Dosis 30 bis 400 mg des Antikörpers beträgt.

**16.** Verfahren nach einem der Ansprüche 11 bis 15, wobei der Antikörper ein Anti-CD4-Antikörper ist.

**17.** Verfahren nach einem der Ansprüche 11 bis 15, wobei der Antikörper ein Anti-CD8-Antikörper ist.

## Revendications

### Revendications pour les Etats contractants suivants : AT, BE, CH, LI, DE, DK, FR, GB, IT, LU, NL, SE

**1.** Utilisation d'un anticorps monoclonal anti-CD4 non-déplétant, c'est-à-dire d'un anticorps qui provoque une déplétion de moins de 50% des cellules T de CD4+ de la périphérie, telle que mesurée par les modifications du nombre des lymphocytes de sang périphérique, pour la préparation d'un médicament pour l'induction d'un état de tolérance immunologique pour un antigène par une méthode qui comprend l'administration dudit anticorps monoclonal anti-CD4 non-déplétant à un sujet, conjointement avec un anticorps monoclonal anti-CD8 non-déplétant, c'est-à-dire un anticorps provoquant une déplétion de moins de 50% des cellules T de CD8+ de la périphérie, telle que mesurée par les modifications des nombres de lymphocytes de sang périphérique, pour induire un environnement permissif de tolérance immunologique dans ledit sujet au moyen desdits anticorps en présence dudit antigène.

**2.** Utilisation d'un anticorps monoclonal anti-CD8 non-déplétant, c'est-à-dire d'un anticorps qui provoque une déplétion de moins de 50% des cellules T de CD8+ de la périphérie, telle que mesurée par les modifications du nombre des lymphocytes de sang périphérique, pour la préparation d'un médicament pour l'induction d'un état de tolérance immunologique pour un antigène par une méthode qui comprend l'administration dudit anticorps monoclonal anti-CD8 non-déplétant à un sujet, conjointement avec un anticorps monoclonal anti-CD4 non-déplétant, c'est-à-dire un anticorps provoquant une déplétion de moins de 50% des cellules T de CD4+ de la périphérie, telle que mesurée par les modifications des nombres de lymphocytes de sang périphérique, pour induire un environnement permissif de tolérance dans ledit sujet au moyen desdits anticorps en présence dudit antigène.

**3.** Utilisation selon l'une des revendications 1 ou 2, caractérisée en ce que ledit antigène est choisi parmi les antigènes de transplantation, les auto-antigènes et les immunoglobulines.

**4.** Utilisation d'un anticorps monoclonal anti-CD4 non-déplétant, c'est-à-dire d'un anticorps qui provoque une déplétion de moins de 50% des cellules T de CD4+ de la périphérie, telle que mesurée par les modifications du nombre des lymphocytes de sang périphérique, en tant qu'anticorps total pour la préparation d'un médicament pour l'induction d'un état de tolérance immunologique pour un antigène choisi parmi les auto-antigènes, les antigènes de transplantation et les immunoglobulines, par une méthode qui comprend l'administration dudit anticorps monoclonal anti-CD4 non-déplétant à un sujet pour induire un environnement permissif de tolérance immunologique dans ledit sujet au moyen dudit anticorps en présence dudit antigène.

**5.** Utilisation selon l'une quelconque des revendications 1 à 4, caractérisée en ce que ladite méthode comprend l'administration dudit anticorps ou desdits anticorps conjointement avec une ou plusieurs thérapies immunosuppressives.

**6.** Utilisation selon l'une quelconque des revendications 1 à 4, caractérisée en ce que ladite méthode comprend l'administration d'un anticorps monoclonal anti-CD4 provoquant une déplétion et/ou d'un anticorps monoclonal anti-CD8 provoquant une déplétion avant l'administration dudit anticorps ou desdits anticorps non-déplétants.

7. Utilisation selon l'une quelconque des revendications 1 à 6, dans laquelle l'anticorps anti-CD4 non-déplétant et, s'il est utilisé, l'anticorps anti-CD8 non-déplétant sont chacun des anticorps recombinants.

8. Utilisation selon l'une quelconque des revendications 1 à 7, dans laquelle l'antigène est un auto-antigène.

9. Utilisation selon la revendication 8, dans laquelle le médicament est destiné à l'induction de tolérance dans le traitement de la polyarthrite rhumatoïde ou de la sclérose en plaques.

10. Utilisation selon l'une quelconque des revendications 1 à 9, dans laquelle l'anticorps anti-CD4 non-déplétant et, s'il est utilisé, l'anticorps anti-CD8 non-déplétant sont administrés chacun à une dose de 1 à 400 mg, 1 à 7 fois par semaine.

11. Composition pharmaceutique comprenant de 1 à 400 mg par dose d'un anticorps anti-CD4 non-déplétant, c'est-à-dire d'un anticorps provoquant une déplétion de moins de 50% des cellules T de CD4+ de la périphérie, telle que mesurée par les modifications du nombre des lymphocytes de sang périphérique, ou d'un anticorps anti-CD8 non-déplétant, c'est-à-dire d'un anticorps provoquant une déplétion de moins de 50% des cellules T de CD8+ de la périphérie, telle que mesurée par les modifications des nombres de lymphocytes de sang périphérique, conjointement avec un support ou diluant pharmaceutiquement acceptable sous une forme appropriée pour une administration à un sujet humain.

12. Composition pharmaceutique selon la revendication 11, dans laquelle la dose est de 3 à 30 mg de l'anticorps.

13. Composition pharmaceutique selon la revendication 12, dans laquelle la dose est de 5 à 20 mg de l'anticorps.

14. Composition pharmaceutique selon la revendication 11, dans laquelle la dose est de 20 à 400 mg de l'anticorps.

15. Composition pharmaceutique selon la revendication 14, dans laquelle la dose est de 30 à 400 mg de l'anticorps.

16. Composition pharmaceutique selon l'une quelconque des revendications 11 à 15, dans laquelle l'anticorps est un anticorps anti-CD4.

17. Composition pharmaceutique selon l'une quelconque des revendications 11 à 15, dans laquelle l'anticorps est un anticorps anti-CD8.


**Revendications pour l'Etat contractant suivant : ES**

1. Utilisation d'un anticorps monoclonal anti-CD4 non-déplétant, c'est-à-dire d'un anticorps qui provoque une déplétion de moins de 50% des cellules T de CD4+ de la périphérie, telle que mesurée par les modifications du nombre des lymphocytes de sang périphérique, pour la préparation d'un médicament pour l'induction d'un état de tolérance immunologique pour un antigène par une méthode qui comprend l'administration dudit anticorps monoclonal anti-CD4 non-déplétant à un sujet, conjointement avec un anticorps monoclonal anti-CD8 non-déplétant, c'est-à-dire un anticorps provoquant une déplétion de moins de 50% des cellules T de CD8+ de la périphérie, telle que mesurée par les modifications des nombres de lymphocytes de sang périphérique, pour induire un environnement permissif de tolérance immunologique dans ledit sujet au moyen desdits anticorps en présence dudit antigène.

2. Utilisation d'un anticorps monoclonal anti-CD8 non-déplétant, c'est-à-dire d'un anticorps qui provoque une déplétion de moins de 50% des cellules T de CD8+ de la périphérie, telle que mesurée par les modifications du nombre des lymphocytes de sang périphérique, pour la préparation d'un médicament pour l'induction d'un état de tolérance immunologique pour un antigène par une méthode qui comprend l'administration dudit anticorps monoclonal anti-CD8 non-déplétant à un sujet, conjointement avec un anticorps monoclonal anti-CD4 non-déplétant, c'est-à-dire un anticorps provoquant une déplétion de moins de 50% des cellules T de CD4+ de la périphérie, telle que mesurée par les modifications des nombres de lymphocytes de sang périphérique, pour induire un environnement permissif de tolérance dans ledit sujet au moyen desdits anticorps en présence dudit antigène.

3. Utilisation selon l'une des revendications 1 ou 2, caractérisée en ce que ledit antigène est choisi parmi les antigènes de transplantation, les auto-antigènes et les immunoglobulines.

4. Utilisation d'un anticorps monoclonal anti-CD4 non-déplétant, c'est-à-dire d'un anticorps qui provoque une déplétion de moins de 50% des cellules T de CD4+ de la périphérie, telle que mesurée par les modifications du nombre des lymphocytes de sang périphérique, en tant qu'anticorps total pour la préparation d'un médicament pour l'induction d'un état de tolérance immunologique pour un antigène choisi parmi les auto-antigènes, les antigènes de transplantation et les immunoglobulines, par une méthode qui comprend l'administration dudit anticorps monoclonal anti-CD4 non-déplétant à un sujet pour induire un environnement permissif de tolérance immunologique dans ledit sujet au moyen dudit anticorps en présence dudit antigène.

5. Utilisation selon l'une quelconque des revendications 1 à 4, caractérisée en ce que ladite méthode comprend l'administration dudit anticorps ou desdits anticorps conjointement avec une ou plusieurs thérapies immunosuppressives.

6. Utilisation selon l'une quelconque des revendications 1 à 4, caractérisée en ce que ladite méthode comprend l'administration d'un anticorps monoclonal anti-CD4 provoquant une déplétion et/ou d'un anticorps monoclonal anti-CD8 provoquant une déplétion avant l'administration dudit anticorps ou desdits anticorps non-déplétants.

7. Utilisation selon l'une quelconque des revendications 1 à 6, dans laquelle l'anticorps anti-CD4 non-déplétant et, s'il est utilisé, l'anticorps anti-CD8 non-déplétant sont chacun des anticorps recombinants.

8. Utilisation selon l'une quelconque des revendications 1 à 7, dans laquelle l'antigène est un auto-antigène.

9. Utilisation selon la revendication 8, dans laquelle le médicament est destiné à l'induction de tolérance dans le traitement de la polyarthrite rhumatoide ou de la sclérose en plaques.

10. Utilisation selon l'une quelconque des revendications 1 à 9, dans laquelle l'anticorps anti-CD4 non-déplétant et, s'il est utilisé, l'anticorps anti-CD8 non-déplétant sont administrés chacun à une dose de 1 à 400 mg, 1 à 7 fois par semaine.

11. Procédé pour la production d'une composition pharmaceutique comprenant le traitement d'un anticorps anti-CD4 non-déplétant, c'est-à-dire d'un anticorps provoquant une déplétion de moins de 50% des cellules T de CD4+ de la périphérie, telle que mesurée par les modifications du nombre des lymphocytes de sang périphérique, ou d'un anticorps anti-CD8 non-déplétant, c'est-à-dire d'un anticorps provoquant une déplétion de moins de 50% des cellules T de CD8+ de la périphérie, telle que mesurée par les modifications des nombres de lymphocytes de sang périphérique, conjointement avec un support ou diluant pharmaceutiquement acceptable pour former une composition appropriée pour une administration à un sujet humain et comprenant de 1 à 400 mg par dose de l'anticorps.

12. Procédé selon la revendication 11, dans lequel la dose est de 3 à 30 mg de l'anticorps.

13. Procédé selon la revendication 12, dans lequel la dose est de 5 à 20 mg de l'anticorps.

14. Procédé selon la revendication 11, dans lequel la dose est de 20 à 400 mg de l'anticorps.

15. Procédé selon la revendication 14, dans lequel la dose est de 30 à 400 mg de l'anticorps.

16. Procédé selon l'une quelconque des revendications 11 à 15, dans lequel l'anticorps est un anticorps anti-CD4.

17. Procédé selon l'une quelconque des revendications 11 à 15, dans lequel l'anticorps est un anticorps anti-CD8.

# Fig.1.

(A)

(B)

Fig.2.

Fig.3.

Fig.4.

## *Fig.5.*

BALB/c→CBA/Ca

*Fig.9*

# Fig.6.

B10BR—►CBA

% SURVIVING SKIN GRAFTS

TIME FROM GRAFTING (DAYS)

EP 0 474 691 B1

Fig. 7

GRAFT SURVIVAL (%)

REGRAFT

>250 DAYS

TIME (DAYS)

28

EP 0 474 691 B1

*Fig.8a.*

*Fig.8b.*

*Fig.*10*a.*

*Fig.*10*b.*

Fig.10c.

Fig.10d.

*Fig.*11a.

*Fig.*11b.